# EUROPÄISCHE PATENTSCHRIFT

(11) **EP 1 272 486 B1**
(45) Veröffentlichungstag und Bekanntmachung des Hinweises auf die Patenterteilung: **11.08.2004**
(21) Anmeldenummer: 01915349.3
(22) Anmeldetag: 15.03.2001
(51) Int. Cl.: C07D 413/04, C07D 231/16, C07D 231/18, C07D 413/14, A01N 43/56, A01N 43/76

(54) **VERFAHREN ZUR HERSTELLUNG VON 7-(PYRAZOL-3-YL)BENZOXAZOLEN**
METHOD FOR PRODUCING 7-(PYRAZOLE-3-YL) BENZOXAZOLES
PROCEDE POUR LA PRODUCTION DE 7-(PYRAZOL-3-YL)BENZOXAZOLES

(30) Priorität: 16.03.2000 DE 10012804
(43) Veröffentlichungstag der Anmeldung: 08.01.2003
(73) Patentinhaber: BASF AKTIENGESELLSCHAFT, 67056 Ludwigshafen (DE)
(72) Erfinder: ZAGAR, Cyrill, 68167 Mannheim (DE); REINHARD, Robert, 67061 Ludwigshafen (DE); PUHL, Michael, 68623 Lampertheim (DE); VOLK, Thorsten, 64297 Darmstadt (DE); GÖTZ, Norbert, 67547 Worms (DE); HAMPRECHT, Gerhard, 69469 Weinheim (DE); MENKE, Olaf, 67317 Altleiningen (DE); SAGASSER, Ingo, 67125 Dannstadt-Schauernheim (DE)
(74) Vertreter: Pohl, Michael, Dr.
(86) Internationale Anmeldenummer: PCT/EP2001/002947
(87) Internationale Veröffentlichungsnummer: WO 2001/068644

(56) Entgegenhaltungen:
- WO-A-96/02138
- WO-A-98/27090
- WO-A-99/55702
- US-A- 5 945 382
- T. MINAMI ET. AL.: "Copper (I) Salt Mediated Arylation of Phosphinyl Stabilised Carbanions and Synthetic Application to Heterocyclic Compounds." JOURNAL OF ORGANIC CHEMISTRY, Bd. 58, Nr. 25, 3. Dezember 1993 (1993-12-03), Seiten 7009-15, XP001005363
- R. J. PERRY ET. AL.: "2-Arylbenzoxazole Formation Through ortho-Fluoro Displacement Reactions." JOURNAL OF ORGANIC CHEMISTRY, Bd. 57, Nr. 23, 6. November 1992 (1992-11-06), Seiten 6351-4, XP001005503

## Beschreibung

Die Erfindung betrifft ein Verfahren zur Herstellung von 7-(Pyrazol-3-yl)benzoxazolen der allgemeinen Formel I 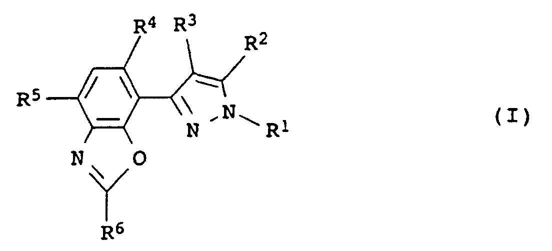 in der die Variablen R¹ - R⁶ folgende Bedeutungen haben:
- R¹: Wasserstoff, C₁-C₄-Alkyl oder C₁-C₄-Halogenalkyl;
- R²: Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
- R³: Wasserstoff, Halogen, Cyano, C₁-C₄-Alkyl oder C₁-C₄-Halogenalkyl;
- R⁴: Halogen;
- R⁵: Fluor, Chlor oder Cyano;
- R⁶: Wasserstoff, C₁-C₆-Alkyl, C₁-C₆-Halogenalkyl, C₂-C₆-Alkenyl, C₂-C₆-Halogenalkenyl, C₂-C₆-Alkinyl, C₁-C₄-Alkoxy-C₁-C₄-alkyl, C₁-C₄-Cyanoalkyl, C₁-C₄-Alkylthio-C₁-C₄-alkyl, (C₁-C₄-Alkoxy)carbonyl-C₁-C₄-alkyl, C₃-C₈-Cycloalkyl, C₃-C₈-Cycloalkyl-C₁-C₄-alkyl, C₃-C₈-Cycloalkyloxy-C₁-C₄-alkyl, Phenyl, Phenyl-C₁-C₄-alkyl, 4- bis 7-gliedriges Heterocyclyl oder Heterocyclyl-C₁-C₄-alkyl, wobei jeder Cycloalkyl-, Phenyl- und jeder Heterocyclyl-Ring unsubstituiert sein oder ein, zwei oder drei Substituenten tragen kann, die unabhängig voneinander ausgewählt sind unter Cyano, Nitro, Amino, Hydroxy, Carboxy, Halogen, C₁-C₄-Alkyl, C₁-C₄-Halogenalkyl, C₁-C₄-Alkoxy, C₁-C₄-Halogenalkoxy , C₁-C₄-Alkylthio.

7-(Pyrazol-3-yl)-benzoxazole der allgemeinen Formel I sind aus der WO 98/27090 und der WO 99/55702 bekannt. Bei diesen Verbindungen handelt es sich um hochwirksame Herbizide.

Im Stand der Technik wird ihre Herstellung ausgehend von den 3-(Pyrazol-3-yl)anilinen der Formel III beschrieben 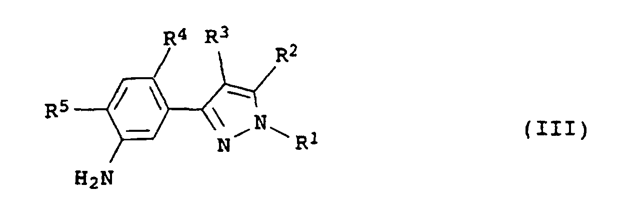 wobei man zunächst die Aminogruppe im Anilin III über eine Diazonium-Zwischenstufe in ein Azidgruppe überführt. Dieses Azid wird anschließend mit einer Carbonsäure R⁶-COOH zum Benzoxazol der allgemeinen Formel I umgesetzt. Dabei kann man zum einen das Azid direkt mit der Carbonsäure zum Benzoxazol umsetzen. Alternativ kann man das Azid mit einer organischen Sulfonsäure über eine Schwefelsäureester-Zwischenstufe zum entsprechenden 2-Hydroxy-3-pyrazolylanilin umsetzen, das dann mit einer Carbonsäure R⁶-COOH oder einem Derivat davon zum Benzoxazol cyclisiert wird.

Diese Herstellungsverfahren sind sehr problematisch, da sich Azide explosionsartig zersetzen können (siehe beispielsweise Houben-Weyl, Methoden der organischen Chemie, Bd. 10/3, Georg-Thieme Verlag Stuttgart, 1965, S.782). Außerdem führt im vorliegenden Fall die Umsetzung des Azids in nicht befriedigenden Ausbeuten und unter Bildung zahlreicher Nebenprodukte zur Zielverbindung I. Die Abtrennung der Nebenprodukte ist aufwendig und teilweise problematisch oder nicht möglich.

G.A. Kraus et al., Tetrahedron Vol. 41, 1985, 2337-2340 beschreiben die Herstellung von 3,5-Dibromorthochinondiazid aus einem Benzoxazol-Derivat. Das Benzoxazol-Derivat wird durch Umsetzung von 2,4,6-Tribromacetanilid in Gegenwart von Natriumhydrid und einem Überschuß an Kupferbromid in Hexamethylphosphorsäuretriamid hergestellt.

W.R. Bowman et al., Tetrahedron Letters, Vol. 23, 1982, 5093-5096 beschreiben im Rahmen ihrer Untersuchungen zur Umwandlung von Thiocarbonsäureaniliden in Benzothiazole auch die Umsetzung von ortho-Iodbenzanilid zu 2-Phenyl-1,3-benzoxazol in Gegenwart von Kupferiodid.

T. Minami et al., J. Org. Chem. 1993, 58, 7009-7015 beobachteten bei der Umsetzung von N-(2-Iodphenyl)-N-methyl-α-(diethoxyphosphinyl)acetamid in Gegenwart äquimolarer Mengen Natriumhydrid und einem doppelt molaren Überschuß an Kupferiodid die Bildung von 2-[(Diethoxyphosphinyl)methyl]benzoxazol.

Keines der vorgenannten Verfahren beschreibt die Herstellung heterocyclisch substituierter Benzoxazole. Die Anzahl der Substituenten am Phenylring des Benzoxazols ist auf zwei beschränkt.

Der vorliegenden Erfindung liegt die Aufgabe zugrunde, ein Verfahren zur Herstellung von 7-(Pyrazol-3-yl)benzoxazolen der allgemeinen Formel I bereitzustellen, bei dem eine Azid-Zwischenstufe vermieden wird.

Diese Aufgabe wird überraschenderweise gelöst durch ein Verfahren, bei dem man ein 2-Halogen-3-(pyrazol-3-yl)anilid der allgemeinen Formel II, 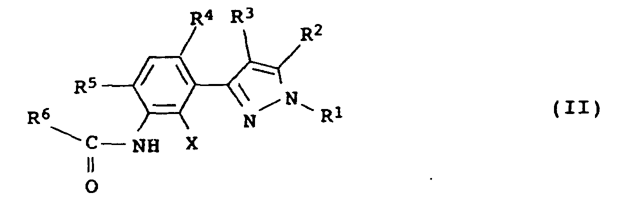 in der die Variablen R¹ - R⁶ die zuvor angegebene Bedeutung haben und X für Brom oder Iod steht, mit einer Base in Gegenwart einer Kupfer(I)-Verbindung umsetzt, wobei das Molverhältnis von Kupfer(I)-Verbindung zu eingesetzter Verbindung (II) < 1:1 ist.

Demnach betrifft die vorliegende Erfindung ein Verfahren zur Herstellung von 7-(Pyrazol-3-yl)benzoxazolen der allgemeinen Formel I wie vorstehend definiert, das dadurch gekennzeichnet ist, dass man ein 2-Halogen-3-(pyrazol-3-yl)anilid der allgemeinen Formel II wie vorstehend definiert mit einer Base in Gegenwart einer Kupfer(I)-Verbindung zu einer Verbindung der allgemeinen Formel I umsetzt, wobei das Molverhältnis von Kupfer(I)-Verbindung zu eingesetzter Verbindung (II) < 1:1 ist.

Die bei der Definition der Substituenten R¹, R², R³, R⁴, R⁵ und R⁶ oder als Reste an Cycloalkyl-, Phenyl- oder heterocyclischen Ringen genannten organischen Molekülteile stellen Sammelbegriffe für individuelle Aufzählungen der einzelnen Gruppenmitglieder dar. Sämtliche Kohlenstoffketten, also alle Alkyl-, Halogenalkyl-, Phenylalkyl-, Cycloalkylalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, Alkenyl-, Halogenalkenyl- und Alkinyl-Gruppen sowie entsprechende Gruppenteile in größeren Gruppen wie Alkoxycarbonyl, Phenylalkyl-, Cycloalkylalkyl, Alkoxycarbonylalkyl etc. können geradkettig oder verzweigt sein, wobei das Präfix Cₙ-Cₘ jeweils die mögliche Anzahl von Kohlenstoffatomen in der Gruppe angibt. Halogenierte Substituenten tragen vorzugsweise ein, zwei, drei, vier oder fünf gleiche oder verschiedene Halogenatome. Die Bedeutung Halogen steht hierbei jeweils für Fluor, Chlor, Brom oder Iod.

Ferner stehen beispielsweise:
- C₁-C₄-Alkyl für: CH₃, C₂H₅, n-Propyl, CH(CH₃)₂, n-Butyl, CH(CH₃)-C₂H₅, CH₂-CH(CH₃)₂ und C(CH₃)₃;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂F, CHF₂, CF₃, CH₂Cl, Dichlormethyl, Trichlormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Alkyl für: C₁-C₄-Alkyl wie vorstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise Methyl, Ethyl, n-Propyl, 1-Methylethyl, n-Butyl, 1,1-Dimethylethyl, n-Pentyl oder n-Hexyl;
- C₁-C₆-Halogenalkyl für: einen C₁-C₆-Alkylrest wie vorstehend genannt, der partiell oder vollständig durch Fluor, Chlor, Brom und/oder Iod substituiert ist, also z.B. einen der unter C₁-C₄-Halogenalkyl genannten Reste sowie für 5-Fluor-1-pentyl, 5-Chlor-1-pentyl, 5-Brom-1-pentyl, 5-Iod-1-pentyl, 5,5,5-Trichlor-1-pentyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl;
- Phenyl-C₁-C₄-alkyl für: Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl oder 1-(Phenylmethyl)-prop-1-yl, vorzugsweise Benzyl oder 2-Phenylethyl;
- Heterocyclyl-C₁-C₄-alkyl für: Heterocyclylmethyl, 1-Heterocyclyl-ethyl, 2-Heterocyclyl-ethyl, 1-Heterocyclyl-prop-1-yl, 2-Heterocyclyl-prop-1-yl, 3-Heterocyclyl-prop-1-yl, 1-Heterocyclyl-but-1-yl, 2-Heterocyclyl-but-1-yl, 3-Heterocyclylbut-1-yl, 4-Heterocyclyl-but-1-yl, 1-Heterocyclyl-but-2-yl, 2-Heterocyclyl-but-2-yl, 3-Heterocyclyl-but-2-yl, 3-Heterocyclyl-but-2-yl, 4-Heterocyclyl-but-2-yl, 1-(Heterocyclyl-methyl)-eth-1-yl, 1-(Heterocyclylmethyl)-1-(methyl)-eth-1-yl oder 1-(Heterocyclylmethyl)-prop-1-yl, vorzugsweise Heterocyclylmethyl oder 2-Heterocyclyl-ethyl;
- Cyano-C₁-C₄-alkyl für: CH₂CN, 1-Cyanoethyl, 2-Cyanoethyl, 1-Cyanoprop-1-yl, 2-Cyanoprop-1-yl, 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 4-Cyanobut-2-yl, 1-(CH₂CN)eth-1-yl, 1-(CH₂CN)-1-(CH₃)-eth-1-yl oder 1-(CH₂CN)prop-1-yl;
- C₁-C₄-Alkoxy für: OCH₃, OC₂H₅, n-Propoxy, OCH(CH₃)₂, n-Butoxy, OCH(CH₃)-C₂H₅, OCH₂-CH(CH₃)₂ oder OC(CH₃)₃, vorzugsweise für OCH₃, OC₂H₅ oder OCH(CH₃)₂;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, 2,2,3,3,3-Pentafluorpropoxy, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy, vorzugsweise für OCHF₂, OCF₃, Dichlorfluormethoxy, Chlordifluormethoxy oder 2,2,2-Trifluorethoxy;
- C₁-C₄-Alkylthio für: SCH₃, SC₂H₅, n-Propylthio, SCH(CH₃)₂, n-Butylthio, SCH(CH₃)-C₂H₅, SCH₂-CH(CH₃)₂ oder SC(CH₃)₃, vorzugsweise für SCH₃ oder SC₂H₅;
- C₁-C₄-Halogenalkylthio für: einen C₁-C₄-Alkylthiorest wie vorstehend genannt, der partiell oder vollständig durch Fluor, Chlor, Brom und/oder Iod substituiert ist, also z.B. SCH₂F, SCHF₂, SCH₂Cl, SCH(Cl)₂, SC(Cl)₃, SCF₃, Chlorfluormethylthio, Dichlorfluormethylthio, Chlordifluormethylthio, 2-Fluorethylthio, 2-Chlorethylthio, 2-Bromethylthio, 2-Iodethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, SC₂F₅, 2-Fluorpropylthio, 3-Fluorpropylthio, 2,2-Difluorpropylthio, 2,3-Difluorpropylthio, 2-Chlorpropylthio, 3-Chlorpropylthio, 2,3-Dichlorpropylthio, 2-Brompropylthio, 3-Brompropylthio, 3,3,3-Trifluorpropylthio, 3,3,3-Trichlorpropylthio, SCH₂-C₂F₅, SCF₂-C₂F₅, 1-(CH₂F)-2-fluorethylthio, 1-(CH₂Cl)-2-chlorethylthio, 1-(CH₂Br)-2-bromethylthio, 4-Fluorbutylthio, 4-Chlorbutylthio, 4-Brombutylthio oder SCF₂-CF₂-C₂F₅, vorzugsweise für SCHF₂, SCF₃, Dichlorfluormethylthio, Chlordifluormethylthio oder 2,2,2-Trifluorethylthio;
- C₁-C₄-Alkoxy-C₁-C₄-alkyl für: durch C₁-C₄-Alkoxy - wie vorstehend genannt - substituiertes C₁-C₄-Alkyl, also z.B. für CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise für CH₂-OCH₃, CH₂-OC₂H₅, 2-Methoxyethyl oder 2-Ethoxyethyl;
- C₁-C₄-Alkylthio-C₁-C₄-alkyl für: durch C₁-C₄-Alkylthio - wie vorstehend genannt - substituiertes C₁-C₄-Alkyl, also z.B. für CH₂-SCH₃, CH₂-SC₂H₅, n-Propylthiomethyl, CH₂-SCH(CH₃)₂, n-Butylthiomethyl, (1-Methylpropylthio)methyl, (2-Methylpropylthio)methyl, CH₂-SC(CH₃)₃, 2-(Methylthio)ethyl, 2-(Ethylthio)-ethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(Methylthio)propyl, 2-(Ethylthio)propyl, 2-(n-Propylthio)propyl, 2-(1-Methylethylthio)propyl, 2-(n-Butylthio)propyl, 2-(1-Methylpropylthio)propyl, 2-(2-Methylpropylthio)propyl, 2-(1,1-Dimethylethylthio)propyl, 3-(Methylthio)propyl, 3-(Ethylthio)propyl, 3-(n-Propylthio)propyl, 3-(1-Methylethylthio)propyl, 3-(n-Butylthio)propyl, 3-(1-Methylpropylthio)-propyl, 3-(2-Methylpropylthio)propyl, 3-(1,1-Dimethylethylthio)propyl, 2-(Methylthio)butyl, 2-(Ethylthio)butyl, 2-(n-Propylthio)butyl, 2-(1-Methylethylthio)butyl, 2-(n-Butylthio)butyl, 2-(1-Methylpropylthio)butyl, 2-(2-Methylpropylthio)butyl, 2-(1,1-Dimethylethylthio)butyl, 3-(Methylthio)butyl, 3-(Ethylthio)butyl, 3-(n-Propylthio)butyl, 3-(1-Methylethylthio)butyl, 3-(n-Butylthio)butyl, 3-(1-Methylpropylthio)butyl, 3-(2-Methylpropylthio)butyl, 3-(1,1-Dimethylethylthio)butyl, 4-(Methylthio)butyl, 4-(Ethylthio)butyl, 4-(n-Propylthio)butyl, 4-(1-Methylethylthio)butyl, 4-(n-Butylthio)-butyl, 4-(1-Methylpropylthio)butyl, 4-(2-Methylpropylthio)butyl oder 4-(1,1-Dimethylethylthio)butyl, vorzugsweise CH₂-SCH₃, CH₂-SC₂H₅, 2-(SCH₃)ethyl oder 2-(SC₂H₅)ethyl;
- (C₁-C₄-Alkoxy)carbonyl-C₁-C₄-alkyl für: durch (C₁-C₄-Alkoxy)-carbonyl - wie vorstehend genannt - substituiertes C₁-C₄-Alkyl, also z.B. für CH₂-CO-OCH₃, CH₂-CO-OC₂H₅, n-Propoxycarbonylmethyl, CH₂-CO-OCH(CH₃)₂, n-Butoxycarbonylmethyl, CH₂-CO-OCH(CH₃)-C₂H₅, CH₂-CO-OCH₂-CH(CH₃)₂, CH₂-CO-OC(CH₃)₃, 1-(Methoxycarbonyl)ethyl, 1-(Ethoxycarbonyl)ethyl, 1-(n-Propoxycarbonyl)ethyl, 1-(1-Methylethoxycarbonyl)ethyl, 1-(n-Butoxycarbonyl)ethyl, 2-(Methoxycarbonyl)ethyl, 2-(Ethoxycarbonyl)ethyl, 2-(n-Propoxycarbonyl)ethyl, 2-(1-Methylethoxycarbonyl)ethyl, 2-(n-Butoxycarbonyl)ethyl, 2-(1-Methylpropoxycarbonyl)ethyl, 2-(2-Methylpropoxycarbonyl)ethyl, 2-(1,1-Dimethylethoxycarbonyl)ethyl, 2-(Methoxycarbonyl)propyl, 2-(Ethoxycarbonyl)propyl, 2-(n-Propoxycarbonyl)propyl, 2-(1-Methylethoxycarbonyl)propyl, 2-(n-Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3-(Methoxycarbonyl)-propyl, 3-(Ethoxycarbonyl)propyl, 3-(n-Propoxycarbonyl)propyl, 3-(1-Methylethoxycarbonyl)propyl, 3-(n-Butoxycarbonyl)propyl, 3-(1-Methylpropoxycarbonyl)propyl, 3-(2-Methylpropoxycarbonyl)propyl, 3-(1,1-Dimethylethoxycarbonyl)propyl, 2-(Methoxycarbonyl)butyl, 2-(Ethoxycarbonyl)butyl, 2-(n-Propoxycarbonyl)butyl, 2-(1-Methylethoxycarbonyl)butyl, 2-(n-Butoxycarbonyl)butyl, 2-(1-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(n-Propoxycarbonyl)butyl, 3-(1-Methylethoxycarbonyl)butyl, 3-(n-Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)butyl, 4-(Ethoxycarbonyl)-butyl, 4-(n-Propoxycarbonyl)butyl, 4-(1-Methylethoxycarbonyl)-butyl, 4-(n-Butoxycarbonyl)butyl, 4-(1-Methylpropoxycarbonyl)-butyl, 4-(2-Methylpropoxycarbonyl)butyl oder 4-(1,1-Dimethylethoxycarbonyl)butyl, vorzugsweise für CH₂-CO-OCH₃, CH₂-CO-OC₂H₅, 1-(Methoxycarbonyl)ethyl oder 1-(Ethoxycarbonyl)ethyl;
- C₁-C₄-Alkylsulfinyl für: SO-CH₃, SO-C₂H₅, SO-CH₂-C₂H₅, SO-CH(CH₃)₂, n-Butylsulfinyl, SO-CH(CH₃)-C₂H₅, SO-CH₂-CH(CH₃)₂ oder SO-C(CH₃)₃, vorzugsweise für SO-CH₃ oder SO-C₂H₅;
- C₁-C₄-Halogenalkylsulfinyl für: einen C₁-C₄-Alkylsulfinylrest - wie vorstehend genannt - der partiell oder vollständig durch Fluor, Chlor, Brom und/oder Iod substituiert ist, also z.B. SO-CH₂F, SO-CHF₂, SO-CF₃, SO-CH₂Cl, SO-CH(Cl)₂, SO-C(Cl)₃, Chlorfluormethylsulfinyl, Dichlorfluormethylsulfinyl, Chlordifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2-Chlor-2-fluorethylsulfinyl, 2-Chlor-2,2-difluorethylsulfinyl, 2,2-Dichlor-2-fluorethylsulfinyl, 2,2,2-Trichlorethylsulfinyl, SO-C₂F₅, 2-Fluorpropylsulfinyl, 3-Fluorpropylsulfinyl, 2,2-Difluorpropylsulfinyl, 2,3-Difluorpropylsulfinyl, 2-Chlorpropylsulfinyl, 3-Chlorpropylsulfinyl, 2,3-Dichlorpropylsulfinyl, 2-Brompropylsulfinyl, 3-Brompropylsulfinyl, 3,3,3-Trifluorpropylsulfinyl, 3,3,3-Trichlorpropylsulfinyl, SO-CH₂-C₂F₅, SO-CF₂-C₂F₅, 1-(CH₂F)-2-fluorethylsulfinyl, 1-(CH₂Cl)-2-chlorethylsulfinyl, 1-(CH₂Br)-2-bromethylsulfinyl, 4-Fluorbutylsulfinyl, 4-Chlorbutylsulfinyl, 4-Brombutylsulfinyl oder Nonafluorbutylsulfinyl, vorzugsweise für SO-CF₃, SO-CH₂Cl oder 2,2,2-Trifluorethylsulfinyl;
- C₁-C₄-Alkylsulfonyl für: SO₂-CH₃, SO₂-C₂H₅, SO₂-CH₂-C₂H₅, SO₂-CH(CH₃)₂, n-Butylsulfonyl, SO₂-CH(CH₃)-C₂H₅, SO₂-CH₂-CH(CH₃)₂ oder SO₂-C(CH₃)₃, vorzugsweise für SO₂-CH₃ oder SO₂-C₂H₅;
- C₁-C₄-Halogenalkylsulfonyl für: einen C₁-C₄-Alkylsulfonylrest - wie vorstehend genannt - der partiell oder vollständig durch Fluor, Chlor, Brom und/oder Iod substituiert ist, also z.B. SO₂-CH₂F, SO₂-CHF₂, SO₂-CF₃, SO₂-CH₂Cl, SO₂-CH(Cl)₂, SO₂-C(Cl)₃, Chlorfluormethylsulfonyl, Dichlorfluormethylsulfonyl, Chlordifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, SO₂-C₂F₅, 2-Fluorpropylsulfonyl, 3-Fluorpropylsulfonyl, 2,2-Difluorpropylsulfonyl, 2,3-Difluorpropylsulfonyl, 2-Chlorpropylsulfonyl, 3-Chlorpropylsulfonyl, 2,3-Dichlorpropylsulfonyl, 2-Brompropylsulfonyl, 3-Brompropylsulfonyl, 3,3,3-Trifluorpropylsulfonyl, 3,3,3-Trichlorpropylsulfonyl, SO₂-CH₂-C₂F₅, SO₂-CF₂-C₂F₅, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl, vorzugsweise für SO₂-CH₂Cl, SO₂-CF₃ oder 2,2,2-Trifluorethylsulfonyl;
- C₂-C₆-Alkenyl für: Ethenyl, Prop-1-en-1-yl, Allyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methylbut-1-en-1-yl, 2-Methylbut-1-en-1-yl, 3-Methylbut-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methylbut-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methylpent-1-en-1-yl, 3-Methyl-pent-1-en-1-yl, 4-Methylpent-1-en-1-yl, 1-Methyl-pent-2-en-1-yl, 2-Methylpent-2-en-1-yl, 3-Methyl-pent-2-en-1-yl, 4-Methylpent-2-en-1-yl, 1-Methyl-pent-3-en-1-yl, 2-Methylpent-3-en-1-yl, 3-Methyl-pent-3-en-1-yl, 4-Methylpent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₂-C₆-Halogenalkenyl für: C₂-C₆-Alkenyl wie vorstehend genannt, das partiell oder vollständig durch Fluor, Chlor, Brom und/oder Iod substituiert ist, also z.B. 2-Chlorethenyl, 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₂-C₆-Alkinyl für: Ethinyl, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methylpent-1-in-1-yl, 3-Methyl-pent-1-in-3-yl, 3-Methylpent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für Prop-2-in-1-yl;
- C₃-C₈-Cycloalkyl für: Cyclopropyl, Cyclobutyl, Cyclopentyl, Cyclohexyl, Cycloheptyl oder Cyclooctyl;
- C₃-C₈-Cycloalkyl-C₁-C₄-alkyl für: Cyclopropylmethyl, 1-Cyclopropyl-ethyl, 2-Cyclopropyl-ethyl, 1-Cyclopropyl-prop-1-yl, 2-Cyclopropyl-prop-1-yl, 3-Cyclopropyl-prop-1-yl, 1-Cyclopropyl-but-1-yl, 2-Cyclopropyl-but-1-yl, 3-Cyclopropyl-but-1-yl, 4-Cyclopropyl-but-1-yl, 1-Cyclopropyl-but-2-yl, 2-Cyclopropyl-but-2-yl, 3-Cyclopropyl-but-2-yl, 3-Cyclopropyl-but-2-yl, 4-Cyclopropyl-but-2-yl, 1-(Cyclopropylmethyl)-eth-1-yl, 1-(Cyclopropylmethyl)-1-(methyl)-eth-1-yl, 1-(Cyclopropylmethyl)-prop-1-yl, Cyclobutylmethyl, 1-Cyclobutyl-ethyl, 2-Cyclobutyl-ethyl, 1-Cyclobutyl-prop-1-yl, 2-Cyclobutylprop-1-yl, 3-Cyclobutyl-prop-1-yl, 1-Cyclobutyl-but-1-yl, 2-Cyclobutyl-but-1-yl, 3-Cyclobutyl-but-1-yl, 4-Cyclobutylbut-1-yl, 1-Cyclobutyl-but-2-yl, 2-Cyclobutyl-but-2-yl, 3-Cyclobutyl-but-2-yl, 3-Cyclobutyl-but-2-yl, 4-Cyclobutylbut-2-yl, 1-(Cyclobutylmethyl)-eth-1-yl, 1-(Cyclobutylmethyl)-1-(methyl)-eth-1-yl, 1-(Cyclobutylmethyl)-prop-1-yl, Cyclopentylmethyl, 1-Cyclopentyl-ethyl, 2-Cyclopentyl-ethyl, 1-Cyclopentyl-prop-1-yl, 2-Cyclopentyl-prop-1-yl, 3-Cyclopentyl-prop-1-yl, 1-Cyclopentyl-but-1-yl, 2-Cyclopentylbut-1-yl, 3-Cyclopentyl-but-1-yl, 4-Cyclopentyl-but-1-yl, 1-Cyclopentyl-but-2-yl, 2-Cyclopentyl-but-2-yl, 3-Cyclopentyl-but-2-yl, 3-Cyclopentyl-but-2-yl, 4-Cyclopentyl-but-2-yl, 1-(Cyclopentylmethyl)-eth-1-yl, 1-(Cyclopentylmethyl)-1-(methyl)-eth-1-yl, 1-(Cyclopentylmethyl)-prop-1-yl, Cyclohexylmethyl, 1-Cyclohexyl-ethyl, 2-Cyclohexyl-ethyl, 1-Cyclohexylprop-1-yl, 2-Cyclohexyl-prop-1-yl, 3-Cyclohexyl-prop-1-yl, 1-Cyclohexyl-but-1-yl, 2-Cyclohexyl-but-1-yl, 3-Cyclohexylbut-1-yl, 4-Cyclohexyl-but-1-yl, 1-Cyclohexyl-but-2-yl, 2-Cyclohexyl-but-2-yl, 3-Cyclohexyl-but-2-yl, 3-Cyclohexylbut-2-yl, 4-Cyclohexyl-but-2-yl, 1-(Cyclohexylmethyl)-eth-1-yl, 1-(Cyclohexylmethyl)-1-(methyl)-eth-1-yl, 1-(Cyclohexylmethyl)-prop-1-yl, Cycloheptylmethyl, 1-Cycloheptyl-ethyl, 2-Cycloheptyl-ethyl, 1-Cycloheptyl-prop-1-yl, 2-Cycloheptyl-prop-1-yl, 3-Cycloheptyl-prop-1-yl, 1-Cycloheptyl-but-1-yl, 2-Cycloheptyl-but-1-yl, 3-Cycloheptyl-but-1-yl, 4-Cycloheptyl-but-1-yl, 1-Cycloheptyl-but-2-yl, 2-Cycloheptyl-but-2-yl, 3-Cycloheptyl-but-2-yl, 3-Cycloheptyl-but-2-yl, 4-Cycloheptyl-but-2-yl, 1-(Cycloheptylmethyl)-eth-1-yl, 1-(Cycloheptylmethyl)-1-(methyl)-eth-1-yl, 1-(Cycloheptylmethyl)-prop-1-yl, Cyclooctylmethyl, 1-Cyclooctyl-ethyl, 2-Cyclooctyl-ethyl, 1-Cyclooctyl-prop-1-yl, 2-Cyclooctylprop-1-yl, 3-Cyclooctyl-prop-1-yl, 1-Cyclooctyl-but-1-yl, 2-Cyclooctyl-but-1-yl, 3-Cyclooctyl-but-1-yl, 4-Cyclooctylbut-1-yl, 1-Cyclooctyl-but-2-yl, 2-Cyclooctyl-but-2-yl, 3-Cyclooctyl-but-2-yl, 3-Cyclooctyl-but-2-yl, 4-Cyclooctylbut-2-yl, 1-(Cyclooctylmethyl)-eth-1-yl, 1-(Cyclooctylmethyl)-1-(methyl)-eth-1-yl oder 1-(Cyclooctylmethyl)-prop-1-yl, vorzugsweise für Cyclopropylmethyl, Cyclobutylmethyl, Cyclopentylmethyl oder Cyclohexylmethyl. Unter 4- bis 7-gliedrigem Heterocyclyl sind sowohl gesättigte, partiell oder vollständig ungesättigte als auch aromatische Heterocyclen mit einem, zwei oder drei Heteroatomen zu verstehen,
wobei die Heteroatome ausgewählt sind unter Stickstoffatomen, Sauerstoff- und Schwefelatomen. Bevorzugtes Heterocyclyl ist 5-bis 7-gliedrig.

### Beispiele für gesättigte Heterocyclen sind:

Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl, 1,3-Dioxepan-6-yl, 1,3-Dithiepan-2-yl, 1,3-Dithiepan-2-yl, 1,3-Dithiepan-2-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl und Hexahydro-1,4-diazepin-2-yl.

### Beispiele für ungesättigte Heterocyclen sind:

Dihydrofuran-2-yl, 1,2-Oxazolin-3-yl, 1,2-Oxazolin-5-yl, 1,3-Oxazolin-2-yl.

Beispiele für aromatisches Heterocyclyl sind die 5- und 6-gliedrigen aromatischen, heterocyclischen Reste, z.B. Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Pyrimidyl, Furanyl und Thienyl.

Im erfindungsgemäßen Verfahrens verwendet man als Übergangsmetall eine Kupfer(I)-Verbindung, insbesondere ein Kupfer(I)-Halogenid, beispielsweise Kupfer(I)-chlorid, Kupfer(I)-bromid oder Kupfer(I)-iodid.

Im erfindungsgemäßen Verfahren kann man neben der die Cyclisierung von II nach I katalysierenden Kupfer(I)-Verbindung auch einen Cokatalysator einsetzen, bei dem es sich um eine Verbindung handelt, die für Kupfer ein Komplexligand ist. Beispiele für Cokatalysatoren sind Phosphine wie Triphenylphosphin, Tri-o-tolylphosphin, Tri-n-butylphosphin, 1,2-Bis(diphenylphosphino)ethan, 1,3-Bis(diphenylphosphino)propan, Phosphite wie Trimethyl-, Triethyl- oder Triisopropylphosphit, Sulfide wie Dimethylsulfid, sowie Cyanid oder Kohlenmonoxid. Sofern erwünscht setzt man den Cokatalysator in der Regel in wenigstens equimolarer Menge, bezogen auf Kupfer, ein.

Die Kupfer(I)-Verbindungen können auch als Komplexverbindungen, die vorzugsweise einen oder mehrere der vorgenannten Cokatalysatoren als Liganden aufweisen, eingesetzt werden. Beispiele für derartige Verbindungen sind [CuBr(S(CH₃)₂)], [CuI(P(OC₂H₅)₃)], [CuI(P(OCH₃)₃)] und [CuCl(PPh₃)₃].

Die Kupfer(I)-Verbindungen können gewünschtenfalls auch auf einem inerten Trägermaterial immobilisiert sein, beispielsweise auf Aktivkohle, Kieselgel, Aluminiumoxid, oder auf einem unlöslichen Polymer z.B. einem Styrol-Divinylbenzol-Copolymer.

Im erfindungsgemäßen Verfahren setzt man die Kupfer(I)-Verbindungen, bezogen auf die Verbindung II, in substöchiometrischer Menge ein. Das Molverhältnis von Kupfer zu eingesetzter Verbindung II ist dann < 1:1. Besonders bevorzugt liegt das Molverhältnis von Kupfer(I)-Verbindung zu eingesetzter Verbindung II im Bereich von 0,05:1 bis 0,8:1, beispielsweise 0,1:1 bis 0,3:1.

Erfindungsgemäß führt man das Verfahren in Gegenwart einer Base durch. Als Base kommen grundsätzlich alle basischen Verbindungen in Betracht, die in der Lage sind, die Amidgruppe in II zu deprotonieren. Bevorzugt sind Basen wie Alkoholate, Amide, Hydride, Hydroxide, Hydrogencarbonate und Carbonate von Alkalimetallen oder Erdalkalimetallen, insbesondere des Lithiums, Kaliums, Natriums, Caesiums oder Kalziums. Beispiele für geeignete Basen sind die Natrium- oder Kaliumalkoholate des Methanols, des Ethanols, des n-Propanols, des iso-Propanols, des n-Butanols und des tert.-Butanols, weiterhin Natriumhydrid und Kaliumhydrid, Kalziumhydrid, Natriumamid, Kaliumamid, Natriumcarbonat, Kaliumcarbonat, Caesiumcarbonat, Natriumhydrogencarbonat, Kaliumhydrogencarbonat, Natriumhydroxid, Kaliumhydroxid und Lithiumhydroxid. In einer bevorzugten Ausführungsform des erfindungsgemäßen Verfahrens setzt man als Base Natriumhydrid ein. In einer anderen, besonders bevorzugten Ausführungsform des erfindungsgemäßen Verfahrens setzt man als Base Kaliumcarbonat und/oder Kaliumhydrogencarbonat ein. Die Base kann in substöchiometrischer, überstöchiometrischer oder äquimolarer Menge eingesetzt werden. Vorzugsweise setzt man wenigstens eine äquimolare Menge an Base, bezogen auf die Verbindung II ein. Insbesondere liegt das Molverhältnis von Base (gerechnet als Basenäquivalente) zu Verbindung II im Bereich von 1:1 bis 1:5 und besonders bevorzugt im Bereich von 1:1 bis 1:1,5.

Vorzugsweise führt man die Umsetzung von II nach I in einem organischen Lösungsmittel durch. Als Lösungsmittel kommen grundsätzlich alle organischen Lösungsmittel in Betracht, die unter den Reaktionsbedingungen inert sind. Hierbei handelt es sich beispielsweise um Kohlenwasserstoffe wie Hexan oder Toluol, halogenierte Kohlenwasserstoffe wie 1,2-Dichlorethan oder Chlorbenzol, Ether wie Dioxan, Tetrahydrofuran (THF), Methyl-tert.-butylether, Dimethoxyethan, Diethylenglykoldimethylether und Triethylenglykoldimethylether, aprotische polare Lösungsmittel, z.B. organische Amide wie Dimethylformamid (DMF), N-Methylpyrrolidon (NMP), N,N-Dimethylacetamid (DMA), Dimethylsulfoxid (DMSO), organische Nitrile wie Acetonitril oder Propionitril sowie tertiäre Stickstoffbasen, z.B. Pyridin. Selbstverständlich können auch Mischungen der genannten Lösungsmittel eingesetzt werden. Bevorzugt werden aprotische polare Lösungsmittel wie DMSO, DMF, NMP, DMA, Acetonitril, Propionitril, Pyridin, Dimethoxyethan, Diethylenglykoldimethylether und Triethylenglycoldimethylether oder deren Mischungen eingesetzt.

Die Umsetzungstemperatur hängt naturgemäß von der Reaktivität der jeweiligen Verbindung II ab. In der Regel wird die Reaktionstemperatur Raumtemperatur nicht unterschreiten. Vorzugsweise führt man die Umsetzung von II nach I bei Temperaturen unterhalb 200°C durch. Häufig wird man bei erhöhter Temperatur, beispielsweise oberhalb 50°C, insbesondere oberhalb 70°C und besonders bevorzugt oberhalb 100°C arbeiten. Vorzugsweise führt man die Reaktion bei Temperaturen unterhalb 180°C und insbesondere unterhalb 160°C durch.

Die Aufarbeitung des Umsetzungsproduktes zur Gewinnung der Zielverbindung I kann nach den hierfür üblichen Methoden erfolgen. In der Regel wird man zunächst extraktiv aufarbeiten oder das verwendete Lösungsmittel nach üblichen Verfahren, beispielsweise destillativ, entfernen. Man kann auch die Zielverbindung I aus der Reaktionsmischung nach Verdünnen der Reaktionsmischung mit Wasser mit einem flüchtigen organischen Lösungsmittel extrahieren, das seinerseits wieder destillativ entfernt wird. Auch kann man die Zielverbindung durch Zugabe von Wasser aus der Reaktionsmischung ausfällen. Hierbei erhält man ein Rohprodukt, welches das Wertprodukt I enthält. Zur weiteren Reinigung kann man die üblichen Verfahren wie Kristallisation oder Chromatographie, beispielsweise an Aluminiumoxiden oder Kieselgelen anwenden. Ebenfalls ist es möglich die nach dem Verfahren erhältlichen Stoffe an optisch aktiven Adsorbaten zur Gewinnung der reinen Isomere zu chromatographieren.

R³ in Formel II steht vorzugsweise für einen von Wasserstoff verschiedenen Rest. Im erfindungsgemäßen Verfahren werden vorzugsweise solche Verbindungen der Formel II eingesetzt, worin die Variablen R¹ bis R⁶ unabhängig voneinander, vorzugsweise jedoch in Kombination miteinander, die nachstehend angegebenen Bedeutungen aufweisen:
- R¹: Wasserstoff oder C₁-C₄-Alkyl, insbesondere Methyl oder Ethyl;
- R²: Cyano, Difluormethoxy, Trifluormethyl oder Methylsulfonyl;
- R³: Halogen;
- R⁴: Halogen;
- R⁵: Fluor, Chlor oder Cyano;
- R⁶: Wasserstoff, C₁-C₄-Alkyl, C₁-C₄-Halogenalkyl, C₂-C₄-Alkenyl, C₂-C₄-Halogenalkenyl, C₂-C₄-Alkinyl, C₁-C₄-Alkoxy-C₁-C₄-alkyl, C₁-C₄-Alkoxycarbonyl-C₁-C₄-alkyl, C₃-C₈-Cycloalkyl, C₃-C₈-Cycloalkyl-C₁-C₄-alkyl, Phenyl, Phenyl-C₁-C₄-alkyl, 4- bis 7-gliedriges Heterocyclyl oder Heterocyclyl-C₁-C₄-alkyl, wobei der Phenylring, Cycloalkylring und der Heterocyclylring unsubstituiert sein können oder einen oder zwei Substituenten, ausgewählt unter Cyano, Halogen, C₁-C₄-Alkyl, C₁-C₄-Halogenalkyl und C₁-C₄-Alkoxy. Beispiele für bevorzugte Bedeutungen von R⁶ sind in Tabelle 1 angegeben.

Hierunter sind solche Verbindungen II besonders bevorzugt, worin R¹ für Methyl steht. R² steht insbesondere für Trifluormethyl und besonders bevorzugt für Difluormethoxy. R³ steht insbesondere für Chlor oder Brom. R⁴ steht insbesondere für Fluor oder Chlor. R⁵ steht insbesondere für Chlor. R⁶ steht insbesondere für Wasserstoff, C₁-C₄-Alkyl, C₁-C₄-Alkoxy-C₁-C₄-alkyl, C₃-C₈-Cycloalkyl, C₃-C₈-Cycloalkyl-C₁-C₄-alkyl, Phenyl oder Phenyl-C₁-C₄-alkyl.

Beispiele für besonders bevorzugte Verbindungen der allgemeinen Formel II sind die Bromanilide der Formeln IIa und IIc und die Iodanilide der Formeln IIb und IId, in denen R¹ für CH₃, R² für OCHF₂ oder CF₃ und R⁵ für Cl stehen und die Variablen R³, R⁴ und R⁶ jeweils einer Zeile der Tabelle 1 entsprechen (Verbindungen IIa.1-IIa.116, Verbindungen IIb.1-IIb.116, Verbindungen IIc.1-IIc.116 und Verbindungen IId.1-IId.116).

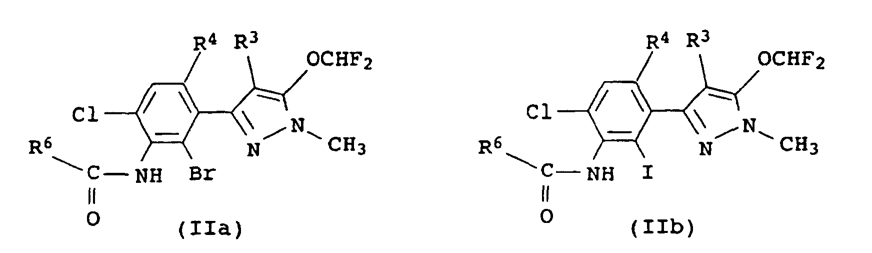

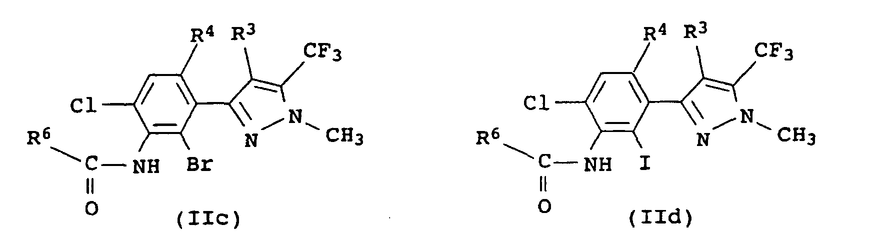

**Tabelle 1**

| Nr. | R³ | R⁴ | R⁶ |
|---|---|---|---|
| 1 | Cl | F | H |
| 2 | Cl | Cl | H |
| 3 | Br | F | H |
| 4 | Br | Cl | H |
| 5 | Cl | F | CH₃ |
| 6 | Cl | Cl | CH₃ |
| 7 | Br | F | CH₃ |
| 8 | Br | Cl | CH₃ |
| 9 | Cl | F | C₂H₅ |
| 10 | Cl | Cl | C₂H₅ |
| 11 | Br | F | C₂H₅ |
| 12 | Br | Cl | C₂H₅ |
| 13 | Cl | F | n-C₃H₇ |
| 14 | Cl | Cl | n-C₃H₇ |
| 15 | Br | F | n-C₃H₇ |
| 16 | Br | Cl | n-C₃H₇ |
| 17 | Cl | F | CH(CH₃)₂ |
| 18 | Cl | Cl | CH(CH₃)₂ |
| 19 | Br | F | CH(CH₃)₂ |
| 20 | Br | Cl | CH(CH₃)₂ |
| 21 | Cl | F | n-C₄H₉ |
| 22 | Cl | Cl | n-C₄H₉ |
| 23 | Br | F | n-C₄H₉ |
| 24 | Br | Cl | n-C₄H₉ |
| 25 | Cl | F | CH₂-CH(CH₃)₂ |
| 26 | Cl | Cl | CH₂-CH(CH₃)₂ |
| 27 | Br | F | CH₂-CH(CH₃)₂ |
| 28 | Br | Cl | CH₂-CH(CH₃)₂ |
| 29 | Cl | F | CH(CH₃)-C₂H₅ |
| 30 | Cl | Cl | CH(CH₃)-C₂H₅ |
| 31 | Br | F | CH(CH₃)-C₂H₅ |
| 32 | Br | Cl | CH(CH₃)-C₂H₅ |
| 33 | Cl | F | C(CH₃)₃ |
| 34 | Cl | Cl | C(CH₃)₃ |
| 35 | Br | F | C(CH₃)₃ |
| 36 | Br | Cl | C(CH₃)₃ |
| 37 | Cl | F | CH₂-Cl |
| 38 | Cl | Cl | CH₂-Cl |
| 39 | Br | F | CH₂-Cl |
| 40 | Br | Cl | CH₂-Cl |
| 41 | Cl | F | CH₂-F |
| 42 | Cl | Cl | CH₂-F |
| 43 | Br | F | CH₂-F |
| 44 | Br | Cl | CH₂-F |
| 45 | Cl | F | CF₃ |
| 46 | Cl | Cl | CF₃ |
| 47 | Br | F | CF₃ |
| 48 | Br | Cl | CF₃ |
| 49 | Cl | F | CH=CH₂ |
| 50 | Cl | Cl | CH=CH₂ |
| 51 | Br | F | CH=CH₂ |
| 52 | Br | Cl | CH=CH₂ |
| 53 | Cl | F | CH=CH-CH₃ |
| 54 | Cl | Cl | CH=CH-CH₃ |
| 55 | Br | F | CH=CH-CH₃ |
| 56 | Br | Cl | CH=CH-CH₃ |
| 57 | Cl | F | CH=CH-Cl |
| 58 | Cl | Cl | CH=CH-Cl |
| 59 | Br | F | CH=CH-Cl |
| 60 | Br | Cl | CH=CH-Cl |
| 61 | Cl | F | C≡CH |
| 62 | Cl | Cl | C≡CH |
| 63 | Br | F | C≡CH |
| 64 | Br | Cl | C≡CH |
| 65 | Cl | F | C≡C-CH₃ |
| 66 | Cl | Cl | C≡C-CH₃ |
| 67 | Br | F | C≡C-CH₃ |
| 68 | Br | Cl | C≡C-CH₃ |
| 69 | Cl | F | CH₂-OCH₃ |
| 70 | Cl | Cl | CH₂-OCH₃ |
| 71 | Br | F | CH₂-OCH₃ |
| 72 | Br | Cl | CH₂-OCH₃ |
| 73 | Cl | F | CH₂-OC₂H₅ |
| 74 | Cl | Cl | CH₂-OC₂H₅ |
| 75 | Br | F | CH₂-OC₂H₅ |
| 76 | Br | Cl | CH₂-OC₂H₅ |
| 77 | Cl | F | CH₂-CH₂-OCH₃ |
| 78 | Cl | Cl | CH₂-CH₂-OCH₃ |
| 79 | Br | F | CH₂-CH₂-OCH₃ |
| 80 | Br | Cl | CH₂-CH₂-OCH₃ |
| 81 | Cl | F | CH₂-CH₂-OC₂H₅ |
| 82 | Cl | Cl | CH₂-CH₂-OC₂H₅ |
| 83 | Br | F | CH₂-CH₂-OC₂H₅ |
| 84 | Br | Cl | CH₂-CH₂-OC₂H₅ |
| 85 | Cl | F | Cyclopropyl |
| 86 | Cl | Cl | Cyclopropyl |
| 87 | Br | F | Cyclopropyl |
| 88 | Br | Cl | Cyclopropyl |
| 89 | Cl | F | Cyclopentyl |
| 90 | Cl | Cl | Cyclopentyl |
| 91 | Br | F | Cyclopentyl |
| 92 | Br | Cl | Cyclopentyl |
| 93 | Cl | F | Cyclohexyl |
| 94 | Cl | Cl | Cyclohexyl |
| 95 | Br | F | Cyclohexyl |
| 96 | Br | Cl | Cyclohexyl |
| 97 | Cl | F | CH₂-Cyclopropyl |
| 98 | Cl | Cl | CH₂-Cyclopropyl |
| 99 | Br | F | CH₂-Cyclopropyl |
| 100 | Br | Cl | CH₂-Cyclopropyl |
| 101 | Cl | F | Phenyl |
| 102 | Cl | Cl | Phenyl |
| 103 | Br | F | Phenyl |
| 104 | Br | Cl | Phenyl |
| 105 | Cl | F | Benzyl |
| 106 | Cl | Cl | Benzyl |
| 107 | Br | F | Benzyl |
| 108 | Br | Cl | Benzyl |
| 109 | Cl | F | Tetrahydrofuran-2-yl |
| 110 | Cl | Cl | Tetrahydrofuran-2-yl |
| 111 | Br | F | Tetrahydrofuran-2-yl |
| 112 | Br | Cl | Tetrahydrofuran-2-yl |
| 113 | Cl | F | Tetrahydrofuran-3-yl |
| 114 | Cl | Cl | Tetrahydrofuran-3-yl |
| 115 | Br | F | Tetrahydrofuran-3-yl |
| 116 | Br | Cl | Tetrahydrofuran-3-yl |

Die Verbindungen der Formel II sind neu und stellen wertvolle Zwischenprodukte bei der Herstellung von Benzoxazolen der Formel I dar. Die Verbindungen II sind daher ebenfalls Gegenstand der vorliegenden Erfindung.

Überraschenderweise wurde gefunden, dass man die Verbindungen II ausgehend von den 3-(Pyrazol-3-yl)anilinen der allgemeinen Formel III in guten Ausbeuten herstellen kann:

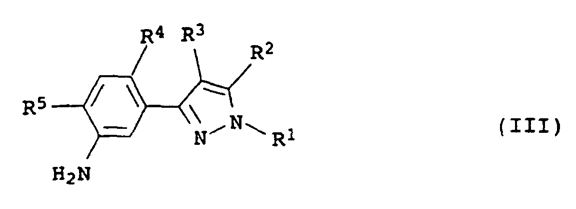

Das Verfahren zur Herstellung der Verbindungen II aus den Verbindungen III umfasst nach einer ersten Variante die folgenden Verfahrensschritte:
i. Halogenierung eines 3-(Pyrazol-3-yl)anilins der Formel III zu einem 2-Halogen-3-(pyrazol-3-yl)anilin der Formel IV, 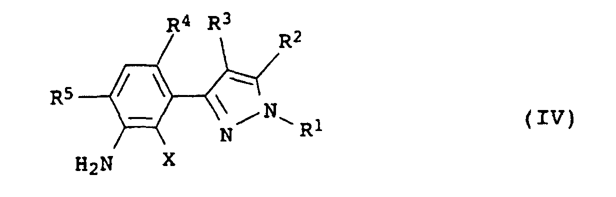
ii. Umsetzung des 3-Halogen-2-(pyrazol-3-yl)anilins IV mit einem Acylierungsmittel der Formel R⁶-C(O)-Y, worin Y für eine Abgangsgruppe steht, zu einem Anilid der Formel II und/oder einer Diacylverbindung der Formel V 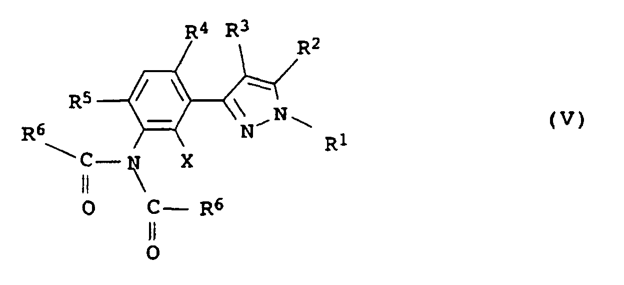
iii.gegebenenfalls partielle Solvolyse der Verbindung V zu dem Anilid der Formel II,
wobei in den Verbindungen der Formeln III, IV und V die Variablen R¹ - R⁶ und X die zuvor genannten Bedeutungen aufweisen. Hinsichtlich bevorzugter und besonders bevorzugter Bedeutungen dieser Variablen gilt das zuvor für die Verbindungen II gesagte. Diese Variante wird insbesondere dann angewendet, wenn R³ von Wasserstoff verschieden ist.

Die 3-Halogen-2-(pyrazol-3-yl)aniline der allgemeinen Formel IV sowie die N,N-Diacyl-3-halogen-2-(pyrazol-3-yl)aniline der allgemeinen Formel V sind ebenfalls neu und stellen wertvolle Zwischenprodukte bei der Herstellung von I aus II dar. Zudem weisen die Verbindungen II, IV und V überraschenderweise eine gute Herbizide Wirksamkeit auf, die zudem der Herbizid-Wirksamkeit von Verbindungen aus dem Stand der Technik, die anstelle des Halogenatoms X ein Wasserstoffatom aufweisen, überlegen. Die Verbindungen II, IV und V sind daher ebenfalls Gegenstand der vorliegenden Erfindung. Für diese Verbindungen gilt hinsichtlich der Variablen R¹ bis R⁶ sowie X das zuvor gesagte.

Dementsprechend sind unter den Verbindungen der allgemeinen Formel IV und V solche Verbindungen besonders bevorzugt, in denen R¹ für Methyl, R² für Difluormethoxy oder für Trifluormethyl und R⁵ für Chlor stehen (Verbindungen IVa und Va (R² = OCHF₂ und X=Br), Verbindungen IVb und Vb (R² = OCHF₂ und X=Iod), Verbindungen IVc und Vc (R² = CF₃ und X=Br), Verbindungen IVd und Vd (R² = CF₃ und X=Iod)).

Beispiele für bevorzugte Verbindungen der allgemeinen Formeln IVa bis IVd sind solche, in denen R³, R⁴ und X jeweils die in einer Zeile der Tabelle 2 angegebene Bedeutung aufweisen (Verbindungen IVa.1-IVa.8, IVb.1-IVb.8, IVc.1-IVc.8, IVd.1-IVd.8).

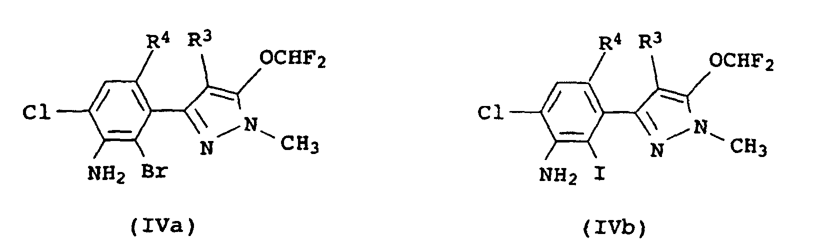

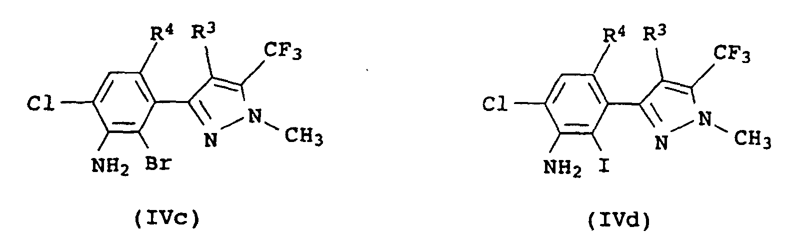

**Tabelle 2**

| Nr. | R³ | R⁴ | X |
|---|---|---|---|
| 1 | Cl | F | Br |
| 2 | Cl | Cl | Br |
| 3 | Br | F | Br |
| 4 | Br | Cl | Br |
| 5 | Cl | F | I |
| 6 | Cl | Cl | I |
| 7 | Br | F | I |
| 8 | Br | Cl | I |

Besonders bevorzugte Verbindungen der allgemeinen Formel Va mit R² = OCHF₂ und X = Br sind solche Verbindungen, in denen R³, R⁴ und R⁶ die in jeweils einer Zeile der Tabelle 1 angegebene Bedeutung aufweisen (Verbindungen Va.1-Va.116). Beispiele für bevorzugte Verbindungen der allgemeinen Formel Vb (R² = OCHF₂ und X = Iod), sind die Verbindungen, in denen R³, R⁴ und R⁶ die in jeweils einer Zeile der Tabelle 1 angegebene Bedeutung aufweisen (Verbindungen Vb.1-Vb.116). Bevorzugte Verbindungen der allgemeinen Formel Vc mit R² = CF₃ und X = Br sind solche Verbindungen, in denen R³, R⁴ und R⁶ die in jeweils einer Zeile der Tabelle 1 angegebene Bedeutung aufweisen (Verbindungen Vc.1-Vc.116). Beispiele für bevorzugte Verbindungen der allgemeinen Formel Vd (R² = CF₃ und X = Iod), sind die Verbindungen, in denen R³, R⁴ und R⁶ die in jeweils einer Zeile der Tabelle 1 angegebene Bedeutung aufweisen (Verbindungen Vd.1-Vd.116).

Geeignete Halogenierungsmittel für die Umwandlung von Verbindungen der Formel III in die 2-Halogen-3-(pyrazol-3-yl)aniline der Formel IV (Schritt i)) sind Brom, Mischungen von Chlor und Brom, Bromchlorid, Iod, Mischungen von Iod und Chlor, Iodchlorid, N-Halogensuccinimide wie N-Bromsuccinimid, N-Iodsuccinimid, Hypohalogensäuren wie Hypobromsäure, weiterhin Dibromisocyanursäure und der Brom-Dioxan-Komplex. Das Halogenierungsmittel wird in der Regel in äquimolarer Menge oder im Überschuß, bezogen auf III, vorzugsweise etwa in der stöchiometrisch benötigten Menge, eingesetzt. Der molare Überschuß kann bis zur 5-fachen Menge von III betragen. Bevorzugt unter den vorgenannten Halogenierungsmitteln sind die Bromierungsmittel und die Iodierungsmittel, wobei in einer bevorzugten Ausführungsform der Erfindung elementares Brom eingesetzt wird.

Gegebenenfalls kann man zur Beschleunigung der Umsetzung i) katalytische oder stöchiometrische Mengen eines Lewis- oder Brönstedsauren Katalysators zusetzen, beispielsweise Aluminiumchlorid oder -bromid, Eisen(III)chlorid oder -bromid, oder Schwefelsäure, oder einen Katalysatorvorläufer, aus dem der eigentliche Katalysator während der Umsetzung gebildet wird, beispielsweise Eisen. Sofern die Verbindung IV als Iodid hergestellt werden soll (X = Iod), kann man als Katalysator auch Salpetersäure, Iodsäure, Schwefeltrioxid, Wasserstoffperoxid oder einen Aluminiumchlorid/ Kupfer(II)chlorid-Komplex einsetzen.

In einer anderen Variante der Umsetzung i) setzt man das gewünschte Halogen in Form eines salzförmigen Halogenids ein, aus dem das Halogen durch Zusatz eines Oxidationsmittels freigesetzt wird. Beispiele für derartige "Halogenierungsmittel" sind Mischungen aus Natriumchlorid oder Natriumbromid mit Wasserstoffperoxid.

Üblicherweise führt man die Halogenierung in einem inerten Lösungsmittel, beispielsweise einem Kohlenwasserstoff wie Hexan, einem halogenierten Kohlenwasserstoff wie Dichlormethan, Trichlormethan, 1,2-Dichlorethan oder Chlorbenzol, in einem cyclischen Ether wie Dioxan, in einer Carbonsäure wie Essigsäure, Propionsäure oder Butansäure, einer Mineralsäure wie Salzsäure oder Schwefelsäure oder in Wasser durch. Selbstverständlich ist es auch möglich, Mischungen der vorgenannten Lösungsmittel zu verwenden.

Gegebenenfalls führt man die Umsetzung in Gegenwart einer Base, beispielsweise einem Alkalimetallhydroxid wie KOH oder dem Alkalimetallsalz einer Carbonsäure wie Natriumacetat oder Natriumpropionat durch.

Die Reaktionstemperatur wird in der Regel durch den Schmelz- und den Siedepunkt des jeweiligen Lösungsmittels bestimmt. vorzugsweise arbeitet man bei Temperaturen im Bereich von 0 bis 100°C und insbesondere im Bereich von 0 bis 80°C.

Das in der Umsetzung i) erhaltene 2-Halogen-3-(pyrazol-3-yl)anilin der Formel IV wird im Schritt ii) mit einem Acylierungsmittel R⁶-C(O)-Y umgesetzt. Hierin hat R⁶ die zuvor genannten Bedeutungen. Y steht für eine übliche Abgangsgruppe.

Beispiele für Acylierungsmittel sind Carbonsäuren (Y = OH), Carbonsäureester wie die C₁-C₄-Alkylester (Y = C₁-C₄-Alkyl, insbesondere Methyl oder Ethyl), Vinylester (Y = CH=CH₂), 2-Propenylester (Y = C(CH₃)=CH₂), die Säureanhydride (Y = O-C(O)-R⁶), Säurehalogenide, insbesondere Säurechloride (Y = Halogen, insbesondere Chlor), Mischungen aus den Anhydriden R⁶-C(O)-O-C(O)-R⁶ mit Carbonsäuren wie Ameisensäure, sowie gemischte Anhydride (Y = O-C(O)-R' mit R' = H oder z.B. C₁-C₆-Alkyl), beispielsweise ein gemischtes Anhydrid mit Pivalinsäure (R' = tert.-Butyl) oder mit Ameisensäure (Verbindungen der Formel H-C(O)-O-C(O)-R⁶).

Das Acylierungsmittel wird vorzugsweise in einer Menge von 1,0 bis 5 Mol und insbesondere in einer Menge von 1,0 bis 2,0 Mol, bezogen auf 1 Mol Verbindung IV eingesetzt.

Gegebenenfalls setzt man bei der Acylierung von IV einen sauren oder basischen Katalysator in katalytischen oder stöchiometrischen Mengen ein. Der Katalysator wird vorzugsweise in einer Menge von 0,001 bis 5 Mol und insbesondere in einer Menge von 0,01 bis 1,2 Mol, bezogen auf 1 Mol Verbindung IV eingesetzt.

Beispiele für basische Katalysatoren sind Stickstoffbasen, z.B. Trialkylamine wie Triethylamin, Pyridinverbindungen wie Pyridin selbst oder Dimethylaminopyridin, weiterhin Oxobasen wie Natriumoder Kaliumcarbonat oder die Hydroxide von Natrium, Kalium oder Calcium.

Beispiele für saure Katalysatoren sind insbesondere Mineralsäuren wie Schwefelsäure.

Üblicherweise führt man die Acylierung in einem Lösungsmittel durch. Geeignete Lösungsmittel sind das gegebenenfalls flüssige Acylierungsmittel selber oder der gegebenenfalls flüssige Katalysator. Geeignete Lösungsmittel sind außerdem inerte organische Lösungsmittel, beispielsweise Kohlenwasserstoffe wie Hexan oder Toluol, halogenierte Kohlenwasserstoffe wie Dichlormethan, Trichlormethan, 1,2-Dichlorethan oder Chlorbenzol, weiterhin Ether wie Dioxan, Tetrahydrofuran, Methyl-tert.-butylether oder Dimethoxyethan.

In einer bevorzugten Ausgestaltung dieses Verfahrensschrittes führt man die Umsetzung von IV in einem flüssigen Anhydrid in Gegenwart konzentrierter Schwefelsäure durch. In einer anderen Ausgestaltung führt man die Umsetzung in einem zweiphasigen System aus Wasser und einem mit Wasser nicht mischbaren organischen Lösungsmittel durch. Diese Ausgestaltung kommt insbesondere dann in Betracht, wenn feste Acylierungsmittel, z.B. feste Säurechloride eingesetzt werden. Als Katalysatoren werden dann häufig basische Katalysatoren, insbesondere anorganische Basen eingesetzt.

In einer weiteren bevorzugten Ausgestaltung dieses Verfahrensschrittes führt man die Umsetzung von IV mit einem Anhydrid (R⁶-CO)₂O oder R⁶-CO-O-CHO oder einer Carbonsäure R⁶-COOH in Gegenwart konzentrierter Schwefelsäure in einem inerten Lösungsmittel durch. In der Regel benötigt man bei dieser Variante geringere Mengen an Acylierungsmitteln, z.B. 1 bis 1,5 Mol, je Mol Verbindung IV. Bei dieser Variante erhält man überraschenderweise mit guten Ausbeuten und hoher Selektivität direkt die Mono-N-acylverbindungen II, ohne dass nennenswerte Mengen der N,N-Diacylverbindungen V gebildet werden.

Bei der Acylierung von IV entsteht neben dem Anilid II häufig auch die Diacylverbindung der allgemeinen Formel V. Je nach Reaktionsführung kann diese auch als alleiniges Reaktionsprodukt anfallen. In diesem Fall wird die Diacylverbindung V, gegebenenfalls in Mischung mit der Verbindung II, einer partiellen Solvolyse unterworfen. Hierbei wird die Verbindung V in die Verbindung II und eine Carbonsäure R⁶-COOH, deren Salz oder ein Derivat, z.B. ein Ester R⁶-COOR' (R' z.B. = C₁-C₄-Alkyl) gespalten.

Als Solvolysemittel kommen beispielsweise Wasser oder Alkohole, beispielsweise C₁-C₄-Alkanole, wie Methanol, Ethanol oder Isopropanol oder Mischungen dieser Alkohole mit Wasser in Betracht.

Vorzugsweise führt man die partielle Solvolyse von V in Gegenwart eines sauren oder basischen Katalysators durch. Beispiele für basische Katalysatoren sind die Alkalimetallhydroxide wie Natriumhydroxid oder Kaliumhydroxid oder die Alkoholate von C₁-C₄-Alkanolen, insbesondere Natrium- oder Kaliummethanolat, oder Natriumoder Kaliumethylat. Beispiele für saure Katalysatoren sind Mineralsäuren wie Salzsäure oder Schwefelsäure.

Üblicherweise setzt man den Solvolysekatalysator in einer Menge von 0,1 bis 5 Mol pro Mol Verbindung V ein. In einer bevorzugten Variante dieses Verfahrensschritts wird der Katalysator in einer Menge von wenigstens 0,5 Mol/Mol Verbindung V und insbesondere etwa äquimolar oder in einem molaren Überschuss, vorzugsweise bis 2 Mol bezogen auf Verbindung V eingesetzt.

Bevorzugte Solvolysemittel sind C₁-C₄-Alkanole. Bevorzugte Katalysatoren sind die Alkalimetallhydroxide oder die Alkalimetall-C₁-C₄-alkoholate wie Natriumhydroxid, Natriummethylat und Natriumethylat.

Üblicherweise führt man die partielle Solvolyse in einem Lösungsmittel durch. Als Lösungsmittel kommen insbesondere die Solvolysemittel selber, beispielsweise die C₁-C₄-Alkanole oder Mischungen dieser Solvolysemittel mit inerten Lösungsmitteln in Betracht. Beispiele für inerte Lösungsmittel sind die vorgenannten Lösungsmittel.

In einer bevorzugten Ausführungsform der vorliegenden Erfindung führt man die Solvolyse von V nach II in einem C₁-C₄-Alkanol in Gegenwart des entsprechenden Alkoholats, vorzugsweise in Methanol oder Ethanol mit Natriummethylat oder Natriumethylat durch.

Die Solvolysetemperatur liegt häufig oberhalb 0°C und wird in der Regel nur durch den Siedepunkt des Lösungsmittels eingeschränkt. Vorzugsweise liegt die Reaktionstemperatur im Bereich von 0 bis 100°C und insbesondere im Bereich von 20 bis 80°C.

Die in den Schritten i), ii) und iii) anfallenden Produkte IV, V und II können nach den hierfür üblichen Aufarbeitungsmethoden isoliert werden. Gegebenenfalls kann man die Reaktionsprodukte der Umsetzung ii) ohne weitere Aufarbeitung in dem Folgeschritt iii) einsetzen. Häufig wird man das bei der Umsetzung ii) bzw. iii) anfallende Rohprodukt der Verbindung II vor der Cyclisierung zum Benzoxazol I einer kristallisativen und/oder chromatographischen Reinigung unterziehen.

Die im erfindungsgemäßen Verfahren als Ausgangsverbindungen eingesetzten 3-(Pyrazol-3-yl)aniline der allgemeinen Formel III sind aus dem Stand der Technik bekannt, beispielsweise aus der WO 98/27090, der WO 99/55702, der WO 92/06962, der WO 92/02509, der WO 96/15115 oder der US 5,032,165, oder können in Analogie zu bekannten Verfahren hergestellt werden. Die Verbindungen der allgemeinen Formel III lassen sich ihrerseits nach den in der WO 92/06962, der WO 92/02509 oder der US 5,032,165 beschriebenen Verfahren ausgehend von Phenyl-substituierten Pyrazolen der Formel VI 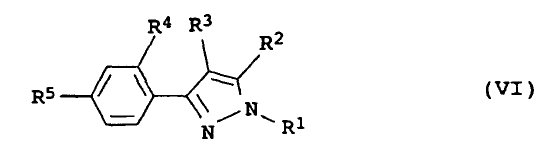 durch sukzessive Nitrierung und Hydrierung der dabei entstandenen Nitrogruppe herstellen.

Die Nitrierung der 3-(Pyrazol-3-yl)benzole VI führt in glatter Reaktion zu den entsprechenden 3-(Pyrazol-3-yl)-1-nitrobenzolen der Formel VII, 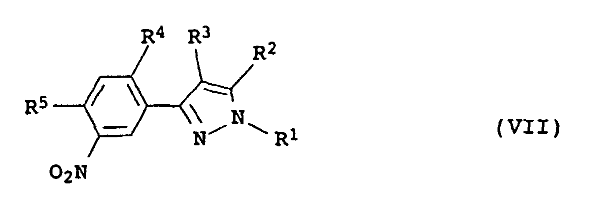 worin R¹ - R⁵ die zuvor genannten Bedeutungen aufweisen. Überraschenderweise gelingt diese Reaktion auch dann, wenn R³ für Wasserstoff steht, ohne dass der Pyrazol-Ring von VI in nennenswertem Umfang nitriert wird. Verbindungen der Formel VI, worin R³ für Wasserstoff steht, werden im Folgenden auch als Verbindungen VI-A bezeichnet. Entsprechendes gilt für die Verbindungen VII.

Die Nitrierung von VI gelingt mit den üblichen Nitrierungsreagenzien, wie sie im Stand der Technik zur Nitrierung von Aromaten sowie in der WO 92/06962, der WO 92/02509 oder der US 5,032,165 beschrieben werden. Geeignete Reagenzien sind Salpetersäure in unterschiedlicher Konzentration, auch konzentrierte und rauchende Salpetersäure, Mischungen von konzentrierter Schwefelsäure und Salpetersäure (Nitriersäure), außerdem Acylnitrate und Alkylnitrate.

Die Nitrierung kann sowohl lösungsmittelfrei, in einem Überschuss des Nitrierreagenzes oder in einem inerten Lösungs- oder Verdünnungsmittel durchgeführt werden, wobei z.B. Wasser, Mineralsäuren, organische Säuren, Halogenkohlenwasserstoffe wie Methylenchlorid, Anhydride wie Essigsäureanhydrid und Mischungen dieser Solventien geeignet sind.

Ausgangsverbindung VI bzw. VI-A und Nitrier-Reagenz können je nach Reagenz in etwa äquimolaren Mengen eingesetzt werden. Zur Optimierung des Umsatzes an Ausgangsverbindung kann es jedoch vorteilhaft sein, das Nitrier-Reagenz im Überschuss zu verwenden, z.B. bis etwa zur 20fachen molaren Menge, bezogen auf VI. Bei der Reaktionsführung ohne Lösungsmittel im Nitrier-Reagenz, z.B. in Nitriersäure, liegt dieses in einem noch größeren Überschuss vor.

Die Reaktionstemperatur liegt normalerweise bei -100°C bis 200°C, bevorzugt bei -30 bis 50°C.

Bevorzugtes Nitrierungsreagenz ist Nitriersäure, d.h. eine Mischung aus konzentrierter Schwefelsäure und konzentrierter, vorzugsweise 100-%iger Salpetersäure. Vorzugsweise löst oder suspendiert man zur Nitrierung die Verbindung VI bzw. VI-A in Schwefelsäure und gibt dann die Salpetersäure, vorzugsweise unter Temperaturkontrolle zu. Die Umsetzung erfolgt dann vorzugsweise bei Temperaturen im Bereich von -30 bis 50°C und vorzugsweise im Bereich von -20 bis + 30°C. Die Reaktionsdauer beträgt in der Regel 0,5 bis 5 h.

Die Isolierung der nitrierten Verbindung VII bzw. VII-A (= Verbindung VII mit R³ = H) aus der Reaktionsmischung erfolgt in üblicher Weise, z.B. durch Gießen des Reaktionsgemischs auf Wasser und/oder Eis und Filtration oder Extraktion des dabei anfallenden Reaktionsprodukts. Gegebenenfalls wird man vor der Isolierung des Reaktionsgemischs die wässrige Phase mit einem Neutralisationsmittel, z.B. Alkalimetallhydroxiden, -carbonaten oder Hydrogencarbonaten abstumpfen. Sofern erforderlich kann das Reaktionsprodukt anschliessend umkristallisiert werden.

Die Reduktion der dabei erhaltenen Nitroverbindungen VII bzw. VII-A zu den 3-(Pyrazol-3-yl)anilinen der allgemeinen Formel III gelingt mit den üblichen Reduktionsmitteln für aromatische Nitrogruppen, wie sie im Stand der Technik zur Reduktion von aromatischen Nitroverbindungen zu den korrespondierenden Anilinen (siehe z.B. J. March, Advanced Organic Chemistry, 3^{rd} ed., J. Wiley & Sons, New-York, 1985, S. 1183 und dort zit. Literatur) sowie in der EP-A 361114, der WO 92/06962, der WO 92/02509 oder der US 5,032,165 beschrieben werden, auf die hiermit Bezug genommen wird.

Die Reduktion gelingt beispielsweise durch Umsetzung der Nitroverbindung VII mit einem Metall wie Eisen, Zink oder Zinn unter sauren Reaktionsbedingungen, d.h. mit naszierendem Wasserstoff, oder mit einem komplexen Hydrid wie Lithiumaluminiumhydrid oder Natriumborhydrid, vorzugsweise in Gegenwart von Übergangsmetallverbindungen des Nickels oder des Kobalts wie NiCl₂(P(Phenyl)₃)₂, oder CoCl₂, (siehe Ono et al. Chem. Ind. (London), 1983 S.480), oder mit NaBH₂S₃ (siehe Lalancette et al. Can. J. Chem. 49, 1971, S. 2990), wobei diese Reduktionen in Abhängigkeit von dem jeweiligen Reagenz in Substanz oder in einem Lösungs- oder Verdünnungsmittel durchgeführt werden kann. Geeignete Lösungsmittel sind abhängig vom Reduktionsmittel z.B. Alkohole wie Methanol, Ethanol, n- und Isopropanol, n-, 2-, Iso- und tert.-Butanol, weiterhin Kohlenwasserstoffe wie Hexan oder Toluol, halogenierte Kohlenwasserstoffe wie Dichlormethan, Trichlormethan, 1,2-Dichlorethan oder Chlorbenzol, weiterhin Ether wie Dioxan, Tetrahydrofuran, Methyl-tert.-butylether oder Dimethoxyethan sowie aliphatische Carbonsäuren und deren Ester, z.B. Essigsäure oder Propionsäure, mit den zuvor genannten C₁-C₄-Alkoholen oder Mischungen der vorgenannten Lösungsmittel.

Bei der Reduktion mit einem Metall arbeitet man vorzugsweise lösungsmittelfrei in einer anorganischen Säure, insbesondere in konzentrierter oder verdünnter Salzsäure, oder in einer flüssigen organischen Säure wie Essigsäure oder Propionsäure. Man kann die Säure jedoch auch mit einem inerten Lösungsmittel, z.B. einem der vorstehend genannten, verdünnen. Die Reduktion mit komplexen Hydriden erfolgt vorzugsweise in einem Lösungsmittel, beispielsweise einem Ether oder einem Alkohol.

Häufig führt man die Reduktion von VII nach III mit Wasserstoff in Gegenwart eines Übergangsmetallkatalysators durch, z.B. mit Wasserstoff in Gegenwart von Katalysatoren auf Basis von Platin, Palladium, Nickel, Ruthenium oder Rhodium. Die Katalysatoren können das Übergangsmetall in elementarer Form oder in Form einer Komplexverbindung, eines Salzes oder eines Oxids des Übergangsmetalls enthalten, wobei man zur Modifizierung der Aktivität übliche Coliganden, z.B. organische Phosphin-Verbindungen wie Triphenylphosphin, Tricyclohexylphosphin, Tri-n-butylphosphine oder Phosphite, einsetzen kann. Der Katalysator wird üblicherweise in Mengen von 0,001 bis 1 Mol, je Mol Verbindung VII eingesetzt, gerechnet als Katalysatormetall.

Der Katalysator kann in geträgerter oder nicht geträgerter Form eingesetzt werden. Geeignete Träger sind beispielsweise Aktivkohle, Calciumcarbonat, Calciumsulfat, Bariumsulfat, Kieselgel, Aluminiumoxid, Alumosilikate, Zeolithe oder organische Polymere, z.B. Popcorn-Polymerisate auf Basis von N-Vinyllactamen oder Polystyrole, die funktionelle, zur Bindung des Katalysatormetalls geeignete funktionelle Gruppen aufweisen. Geeigneter Katalysator ist insbesondere auch fein verteiltes Nickel, z.B. in Form von Raney-Nickel.

Häufig führt man die Umsetzung von VII nach III mit Wasserstoff in Gegenwart eines Übergangsmetallkatalysators in einem inerten organischen Lösungsmittel durch. Geeignet sind grundsätzlich alle der vorgenannten Lösungsmittel. Bevorzugte Lösungsmittel sind die vorgenannten Alkohole und deren Mischungen mit Ethern oder Estern, insbesondere bei Verwendung von fein verteiltem Nickel als Katalysator.

Der zur Reduktion erforderliche Wasserstoffdruck kann über einen weiten Bereich variiert werden und liegt in der Regel im Bereich von Normaldruck bis hin zu einem Überdruck von 50, vorzugsweise bis 10 und insbesondere bis 3 bar. Selbstverständlich gelingt die Umsetzung auch bei Wasserstoffpartialdrucken unterhalb Normaldruck, z.B. im Bereich von 0,2 bis 1 bar.

Die zur Umsetzung erforderliche Temperatur kann über einen weiten Bereich variiert werden und liegt in Abhängigkeit von der Reaktivität des Katalysators und dem gewählten Wasserstoffpartialdruck üblicherweise im Bereich von 0 bis 150°C, vorzugsweise im Bereich von 10 bis 100°C, wobei man die Umsetzung sowohl bei niedrigeren Temperaturen als auch bei höheren Temperaturen durchführen kann.

Nach einer besonderen Ausgestaltung des erfindungsgemässen Verfahrens geht man zur Herstellung der Verbindungen III, in denen X und R³ für Brom oder Iod stehen, direkt von den 3-(Pyrazol-3-yl)benzolen der Formel VI-A aus.

Diese werden in einem ersten Schritt a) mit einem Nitrierungsreagenz zu einem ein 3-(Pyrazol-3-yl)-1-nitrobenzol VII-A 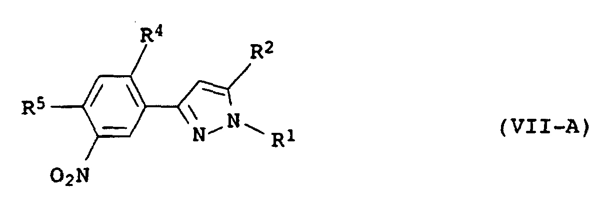 umgesetzt. Anschliessend wird in einem Schritt b die Verbindung VII-A mit einem Reduktionsmittel zu einem 3-(Pyrazol-3-yl)anilin der Formel III-A (Verbindung III mit R³ = H) umsetzt. Die Verbindungen III-A können dann in der oben beschriebenen Weise direkt zu solchen Verbindungen IV bromiert werden, in denen sowohl R³ als auch X für Brom stehen (Verbindungen IV-Br₂), oder iodiert werden zu Verbindungen IV-I₂, in denen sowohl R³ als auch X für Iod stehen.

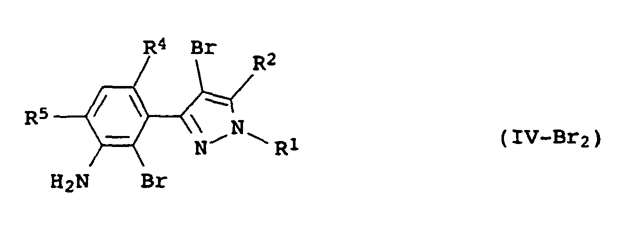

Anschliessend werden die Schritte ii und gegebenenfalls iii in der zuvor beschriebenen Weise durchgeführt.

Die Halogenierung von III-A zu IV gelingt analog der Halogenierung von III zu IV in der oben beschriebenen Art und Weise. Zur Erzielung eines vollständigen Umsatzes wird man im Unterschied zur Halogenierung von III zu IV das Halogenierungsreagenz vorzugsweise in einer Menge von wenigstens etwa 2 Äquivalenten, bezogen auf III-A einsetzen, z.B. im Molverhältnis von 1:1,9 bis 1:2,5, wobei auch ein grösserer Überschuss an Halogenierungsmittel eingesetzt werden kann. Bevorzugtes Halogenierungsmittel für die Umsetzung von III-A nach IV-Br₂ bzw. IV-I₂ ist elementares Halogen, vorzugsweise Brom. Vorzugsweise erfolgt dann die Umsetzung in einer Carbonsäure wie Essigsäure, Propionsäure oder Butansäure oder in einer Mineralsäure wie Salzsäure oder Schwefelsäure, in Mischungen dieser Säuren, in Wasser, oder in einer Mischung wenigstens einer dieser Säuren mit Wasser durch. Die für die Umsetzung erforderlichen Temperaturen kann der Fachmann in einfachen Routineexperimenten ermitteln und liegen bevorzugt im Bereich von - 10°C bis 120°C und insbesondere im Bereich von 10°C bis 60°C.

Die Verbindungen der Formeln II, IV und V und deren landwirtschaftlich brauchbaren Salze eignen sich überraschenderweise - sowohl als Isomerengemische als auch in Form der reinen Isomere - als Herbizide.

Die folgenden Beispiele sollen die Erfindung verdeutlichen, ohne sie jedoch einzuschränken.

Alle ¹H-NMR-Spektren wurden in CDCl₃ in einem 270 MHz-Spektrometer gemessen. Es wurde die chemische Verschiebung gegen TMS in ppm und das Integral des jeweiligen Signals bestimmt. Folgende Abkürzungen wurden verwendet: s = Singulett, d = Dublett, q = Quartett, t = Triplett, br = breites Signal.

### Beispiel 1: Herstellung von 2-Ethyl-4,6-dichlor-7-(4-brom-5-difluormethoxy-1-methyl-1H-pyrazol-3-yl)benzoxazol (1)

1.1 2-Brom-4,6-dichlor-3-(4-brom-5-difluormethoxy-1-methyl-1H-pyrazol-3-yl)anilin IVa.4
   Variante A
      50 g (0,12 mol) 4,6-Dichlor-3-(4-brom-5-difluormethoxy-1-methyl-1H-pyrazol-3-yl)anilin wurden in 200 ml Dichlormethan gelöst und mit 20 g (0,13 mol) Brom versetzt. Die Reaktionsmischung wurde so lange bei Raumtemperatur gerührt, bis sich im HPLC (C₁₈-Säule, Methanol/Wasser-Gradient 0-100) keine weitere Veränderung mehr zeigte. Anschließend extrahierte man die Reaktionsmischung mit gesättigter Natriumhydrogencarbonatlösung und gesättigter Kochsalzlösung. Die organische Phase wurde über Magnesiumsulfat getrocknet und eingeengt. Chromatographie, des Rückstands an Kieselgel (Essigester/Cyclohexan) lieferte 60 g 2-Brom-4,6-dichlor-3-(4-brom-5-difluormethoxy-1-methyl-1H-pyrazol-3-yl)anilin IVa.4.
      - ¹H-NMR δ ppm:: 7,4 (s, 1H), 6,7 (t, 1H), 4,2 (br, 2H) 3,9 (s, 3H)
   Variante B:
      50 g (0,129 mol) 4,6-Dichlor-3-(4-brom-5-difluormethoxy-1-methyl-1H-pyrazol-3-yl)anilin wurden in 150 ml Eisessig gegeben. Hierzu gab man 53 g (0,64 mol) Natriumacetat. Man tropfte bei Raumtempertaur 20,6 g (0,129 mol) Brom zu und rührte die Mischung über Nacht. Man entfernte die Essigsäure im Vakuum, versetzte den Rückstand mit 50 ml Toluol und engte bis zur Trockne ein. Der Rückstand wurde in 200 ml Ethylacetat aufgenommen, mit 100 ml 2 N NaOH-Lösung gewaschen, über-Magensiumsulfat getrocknet und eingeengt. Man erhielt 56 g der Titelverbindung.
1.2 N-Propionyl-2-brom-4,6-dichlor-3-(4-brom-5-difluormethoxy-1-methyl-1H-pyrazol-3-yl)anilid IIa.12
   Variante A
      48 g (0,103 mol) der Verbindung IVa.4 löste man in 480 ml Propionsäureanhydrid und gab hierzu 0,5 g konzentrierte Schwefelsäure. Anschließend rührte man 1 h bei 75°C. Man engte die Reaktionsmischung im Vakuum ein, verdünnte mit Methyl-tert.-butylether und Wasser und trennte die organische Phase ab. Anschließend wusch man die organische Phase mit gesättigter Natriumhydrogencarbonatlösung und trocknete über Magnesiumsulfat. Einengen des Lösungsmittels bis zur Trockene lieferte 57,4 g der Dipropionylverbindung (Verbindung V mit R¹ = Methyl, R² = Difluormethoxy, R³ = Brom, R⁴ = R⁵ = Chlor, X = Brom und R⁶ = Ethyl), die untergeordnete Mengen des entsprechenden N-Propionylanilids IIa.12 enthielt. Die so erhaltene Mischung wurde anschließend einer Partialsolvolyse unterworfen. Hierzu löste man das Reaktionsprodukt der Acylierung in 300 ml Methanol und gab zu der Lösung 17,9 g einer 30 gew.-%igen Lösung von Natriummethylat in Methanol. Anschließend rührte man bei Raumtemperatur, bis sich bei HPLC-Kontrolle (C₁₈-Säule, Methanol/Wasser-Gradient 0-100) keine weitere Umsetzung mehr beobachten ließ. Zur Aufarbeitung gab man zu der Reaktionsmischung etwa 500 ml Dichlormethan und wusch die so erhaltene Lösung nacheinander mit verdünnter Salzsäure, Wasser und gesättigter, wässriger Kochsalzsalzlösung. Nach Chromatographie an Kieselgel (Eluent: Cyclohexan/Ethylacetat) erhielt man 41,4 g N-Propionyl-2-brom-4,6-dichlor-3-(4-brom-5-difluormethoxy-1-methyl-1H-pyrazol-3-yl)anilid IIa.12.
      Schmelzpunkt: 173-175°C
      - ¹H-NMR δ ppm:: 7,6 (s, 1H), 7,2 (br. s, 1H), 6,7 (t, 1H), 3,9 (s, 3H), 2,5 (br. q, 2H), 1,3 (br. t, 3H).

      Die Dipropionylverbindung wies im ¹H-NMR-Spektrum die folgenden Signale auf:
      - ¹H-NMR δ ppm:: 7,7 (s, 1H), 6,7 (t, 1H), 3,9 (s, 3H), 2,8-2,4 (m, 4H), 1,2 (m, 6H).
   Variante B
      56 g (0.12 mol) Anilin IVa.4 aus der vorhergehenden Stufe wurden in 500 ml Toluol gelöst und mit 0,6 g konzentrierter Schwefelsäure und 17,2 g (0,13 mol) Propionsäureanhydrid versetzt. Man rührte 2 d bei Raumtemperatur, saugte den Niederschlag ab, wusch mit wenig Methyl-tert.-butylether nach, nahm den Niederschlag in 300 ml Ethylacetat auf und versetzte mit gerade der Menge 10 %-iger NaOH-Lösung, die nötig *war,* um das Produkt vollständig in Lösung zu bringen. Man trennte die organische Phase ab und isolierte hieraus durch Einengen 49 g der Titelverbindung IIa.12.
1.3 2-Ethyl-4,6-dichlor-7-(4-brom-5-difluormethoxy-1-methyl-1H-pyrazol-3-yl)benzoxazol (1)
   a) Variante mit äquimolarer Menge CuBr:
      2 g des Anilids IIa.12 (0,0038 mol) wurden mit 0,1 g (0,0038 mol) Natriumhydrid (97 gew.-%ig) in 20 ml DMSO bei 70°C umgesetzt, bis keine Gasentwicklung mehr zu beobachten war. Danach gab man 0,55 g (0,0038 mol) Kupfer(I)bromid zu und erwärmte auf 140°C, bis eine HPLC-Kontrolle keine weitere Veränderung mehr zeigte. Man kühlte auf Raumtemperatur ab, gab Eiswasser zu und extrahierte mit etwa 100 ml Ethylacetat. Nach Trocknen über Magnesiumsulfat engte man das Lösungsmittel bis zur Trockene ein und chromatographierte den Rückstand an Kieselgel (Eluent: Cyclohexan/Ethylacetat). Man erhielt auf diese Weise 1,1 g des Benzoxazols (1).
      Schmelzpunkt: 131-132 °C
      - ¹H-NMR δ ppm:: 7,5 (s, 1H), 6,7 (t, 1H), 3,9 (s, 3H), 2,4 (q, 2H), 1,4 (t, 3H)
   b) Variante mit katalytischer Menge CuCl
      3 g (5,7 mmol) Anilid IIa.12 wurden in 10 ml Dimethylformamid und 1 ml Pyridin gelöst. Hierzu gab man 0,43 g (3,1 mmol) K₂CO₃ und erhitzte die Mischung 2 h auf 90°C. Dann gab man 0,12 g (1,2 mmol) Cu(I)Cl zu und rührte 2h bei 140°C. Die Reaktionsmischung wurde im Vakuum eingeengt und an Kieselgel mit Cyclohexan/Ethylacetat 9/1 chromatographiert. Man erhielt 1,9 g des Benzoxazols (1).
   c) Variante mit katalytischer Menge CuBr:
      Analog der vorhergehenden Variante wurden 3 g des Anilids IIa.12 mit 0,3 g (2,3 mmol) Cu(I)Br und 0,43 g (3,1 mmol) K₂CO₃ unter ansonsten identischen Bedingungen umgesetzt. Man erhielt so 1,96 g des Benzoxazols (1).
   d) Variante mit katalytischer Menge Cu(I)I:
      Analog der vorhergehenden Variante wurden 3 g des Anilids IIa.12 mit 0,23 g (1,2 mmol) Cu(I)I und 0,43 g (3,1 mmol) K₂CO₃ unter ansonsten identischen Bedingungen umgesetzt. Man erhielt so 1,8 g des Benzoxazols (1).
   e) Variante mit katalytischer Menge Cu(I)Br und KHCO₃ als Base:
      Analog der vorhergehenden Variante wurden 3 g des Anilids IIa.12 mit 0.63 g (6.3 mmol) KHCO₃ und 0,17 g (1,2 mmol) Cu(I)Br unter ansonsten identischen Bedingungen umgesetzt. Man erhielt 1,8 g des Benzoxazols (1).

### Beispiel 2: Herstellung von 2-Ethyl-4-chlor-6-fluor-7-(4-chlor-2-difluormethoxy-1-methyl-1H-pyrazol-3-yl)benzoxazol (2)

2.1 2-Brom-4-fluor-6-chlor-3-(4-chlor-5-difluormethoxy-1-methyl-1H-pyrazol-3-yl)anilin IVa.1
   Variante A
      57 g (0,165 mol) 4-Fluor-6-chlor-3-(4-chlor-5-difluormethoxy-1-methyl-1H-pyrazol-3-yl)anilin wurden in 300 ml Dichlormethan gelöst und mit 26,5 g (0,165 mol) Brom versetzt. Die Reaktionsmischung wurde so lange bei Raumtemperatur gerührt, bis eine HPLC-Kontrolle (s.o.) keine weitere Veränderung mehr zeigte. Die Reaktionsmischung wurde eingeengt. Man erhielt hierbei 61 g der Verbindung IVa.1.
      - ¹H-NMR δ ppm:: 7,2 (s, 1H), 6,7 (t, 1H), 4,5 (br, 2H) 3,9 (S, 3H)
   Variante B:
      19,5 g (59 mmol) 6-Chlor-4-fluor-3-(4-chlor-5-difluormethoxy-1-methyl-1H-pyrazol-3-yl)anilin wurden in 200 ml Eisessig gegeben. Hierzu gab man 47,8 g (0,59 mol) Natriumacetat. Man tropfte bei Raumtemperatur 10,3 g (64 mmol) Brom zu und rührte die Mischung über Nacht. Man entfernte die Essigsäure im Vakuum, versetzte den Rückstand mit 200 ml Toluol und engte bis zur Trockne ein. Der Rückstand wurde in 200 ml Dichlormethan aufgenommen, mit 200 ml 5-%iger, wässriger NaOH-Lösung gewaschen, über Magnesiumsulfat getrocknet und eingeengt. Man erhielt 24 g der Titelverbindung IVa.1.
2.2 N-Propionyl-2-brom-4-fluor-6-chlor-3-(4-chlor-5-difluormethoxy-1-methyl-1H-pyrazol-3-yl)anilid IIa.9
   Variante A
      43 g (0,106 mol) der Verbindung IVa.1 löste man in 200 ml Propionsäureanhydrid und gab hierzu 0,5 g konzentrierte Schwefelsäure. Anschließend rührte man 1 h bei 75°C. Man engte die Reaktionsmischung im Vakuum ein, verdünnte mit Methyl-tert.-butylether und Wasser und trennte die organische Phase ab. Anschließend wusch man die organische Phase mit gesättigter, wässriger Natriumhydrogencarbonatlösung und trocknete über Magnesiumsulfat. Einengen des Lösungsmittels bis zur Trockene lieferte 47 g der Dipropionylverbindung (Verbindung V mit R¹ = Methyl, R² = Difluormethoxy, R³ = Chlor, R⁴ = Fluor, R⁵ = Chlor, X = Brom und R⁶ = Ethyl), die noch untergeordnete Mengen des entsprechenden N-Propionylanilids IIa.9 enthielt. Die so erhaltene Mischung wurde anschließend einer Partialsolvolyse unterworfen. Hierzu löste man das Reaktionsprodukt der Acylierung in 100 ml Methanol und gab zu der Lösung 32,7 g einer 30 gew.-%igen Lösung von Natriummethylat in Methanol. Anschließend rührte man bei Raumtemperatur, bis eine HPLC-Kontrolle (s.o.) keine weitere Umsetzung mehr zeigte. Zur Aufarbeitung gab man zu der Reaktionsmischung etwa 500 ml Dichlormethan und wusch die so erhaltene Lösung nacheinander mit verdünnter Salzsäure, Wasser und gesättigter, wässriger Kochsalzlösung. Nach Chromatographie an Kieselgel (Eluent: Cyclohexan/Ethylacetat) erhielt man 14 g N-Propionyl-2-brom-4-fluor-6-chlor-3-(4-chlor-5-difluormethoxy-1-methyl-1H-pyrazol-3-yl)anilid IIa.13.
      - ¹H-NMR δ ppm:: 7,6 (br. s, 1H), 7,3 (d, 1H), 6,7 (t, 1H), 3,9 (s, 3H), 2,4 (q, 2H), 1,3 (t, 3H)
   Variante B
      23 g (57 mmol) Anilin IVa.1 wurden in 20 ml Toluol gelöst und mit 0,28 g konzentrierter Schwefelsäure und 8,1 g (63 mmol) Propionsäureanhydrid versetzt. Man rührte 16 h bei Raumtemperatur, verdünnte dann mit 100 ml Wasser und 100 ml Toluol, trennte die Phasen und extrahierte die wässrige Phase nochmals mit insgesamt 200 ml Toluol. Die vereinigten organischen Phasen wurden über Magnesiumsulfat getrocknet und dann eingeengt. Der Rückstand wurde in 50 ml Cyclohexan/Ethylacetat 4:1 (v/v) aufgenommen, erhitzt und dann abgesaugt. Man erhielt 21g der Titelverbindung IIa.13.
2.3 2-Ethyl-4-chlor-6-fluor-7-(4-chlor-5-difluormethoxy-1-methyl-1H-pyrazol-3-yl)benzoxazol (2)
   14 g (0,03 mol) der Verbindung IIa.9 wurden mit 0,75 g (0,03 mol) Natriumhydrid (als 97 gew.-%ige Suspension in Mineralöl) in 50 ml DMSO bei 70°C umgesetzt, bis keine Gasentwicklung mehr zu beobachten war. Danach gab man 0,56 g (0,0039 mol) Kupfer(I)bromid zu und erwärmte auf 140°C, bis eine HPLC-Kontrolle keine weitere Veränderung mehr zeigte. Man kühlte auf Raumtemperatur ab, gab Eiswasser zu und extrahierte mit etwa 100 ml Ethylacetat. Nach Trocknen über Magnesiumsulfat engte man das Lösungsmittel bis zur Trockene ein und chromatographierte den Rückstand an Kieselgel (Eluent: Cyclohexan/Ethylacetat). Man erhielt auf diese Weise 1,1 g des Benzoxazols (6).
   Schmelzpunkt: 73-75 °C
   - ¹H-NMR δ ppm:: 7,3 (s, 1H), 6,7 (t, 1H), 3,9 (s, 3H), 3,0 (q, 2H), 1,44 (t, 3H)

### Beispiel 3: Herstellung von 2-Methyl-4,6-dichlor-7-(4-brom-2-difluormethoxy-1-methyl-1H-pyrazol-3-yl)benzoxazol (3)

3.1 N-Acetyl-2-brom-4,6-dichlor-3-(4-brom-5-difluormethoxy-1-methyl-1H-pyrazol-3-yl)anilid IIa.8
   In Analogie zu Beispiel 1.2 Variante A wurden 5.0 g (0.011 mol) des Anilins IVa.4 zunächst in 100 ml Eisessig unter ansonsten identischen Bedingungen umgesetzt und nachfolgend die primär erhaltene Diacetylverbindung Va.8 mit 8,5 g (0,047 mol) Natriummethanolat in Methanol gespalten. Man erhielt so 5,26 g des Anilids IIa.8.
   ¹H-NMR (CDCl₃) δ(ppm) 7,6 (s, 1 H), 7,1 (br. s. 1 H), 6,7 (t, 1 H), 3,9 (s, 3 H), 2,1 (s, 3 H).
3.2 2-Methyl-4,6-dichlor-7-(4-brom-5-difluormethoxy-1-methyl-1H-pyrazol-3-yl)benzoxazol (3)
   5,2 g (0,01 mol) Anilid IIa.8 wurden in 100 ml Dimethylsulfoxid mit 0,24 g (0,01 mol NaH und 0,22 g (1,5 mmol) Cu(I)Br gemäss der Vorschrift in Beispiel 2.1 umgesetzt. Man erhielt auf diese Weise 1.9 g des Benzoxazols (3).
   ¹H-NMR (CDCl₃) δ(ppm) 7,5 (s, 1 H), 6,8 (t, 1 H), 3,9 (s, 3 H), 2,6 (s, 3 H).

### Beispiel 4: 2-tert-Butyl-4,6-dichlor-7-(4-brom-5-difluormethoxy-1-methyl-1H-pprazol-3-yl)benzoxazol (4)

4.1 N-Pivaloyl-2-brom-4,6-dichlor-3-(4-brom-5-difluormethoxy-1-methyl-1H-pyrazol-3-yl)anilid IIa.36
   3,8 g (8,2 mmol) Anilin IVa.4 wurden in 100 ml Dichlormethan gelöst mit 3,9 g (49,2 mmol) Pyridin und einer katalytischen Menge 4-Dimethylaminopyridin versetzt. Hierzu tropfte man 3,2 g (24,7 mmol) Pivalinsäurechorid.
   Man erwärmte die so erhaltene Mischung auf 40 °C und rührte bis ein Dünnschichtchromatogramm (Cyclohexan/Ethylacetat 4/1 v/v) keine weitere Veränderung mehr zeigte. Man verdünnte mit 200 ml Ethylacetat, wusch zwei mal mit 200 ml 10 %-iger Salzsäure, ein mal mit 100 ml gesättigter NaHCO₃-Lösung und trocknete die organische Phase über Magnesiumsulfat. Durch Chromatographie des nach Einengen erhaltenen Rückstands an Kieselgel (Cyclohexan/Ethylacetat 4/1 v/v) erhielt man 1,4 g des Anilids IIa.36.
   ¹H-NMR (CDCl₃) δ(ppm) 7, 6 (s, 1 H), 7,2 (br, 1 H), 6,7 (t, 1 H) 3,8 (s, 3 H), 1,4 (s, 9 H).
4.2 2-tert.-Butyl-4,6-dichlor-7-(4-brom-5-difluormethoxy-1-methyl-1H-pyrazol-3-yl)benzoxazol (4)
   1,3 g (2,4 mmol) Anilid IIa.36 wurden in 10 ml Dimethylformamid und 1 ml Pyridin gelöst und mit 0,26 g (2,6 mmol) KHCO₃ versetzt. Die Mischung wurde 1,5 h auf 90°C erhitzt. Dann gab man 0,07 g (0,5 mmol) Cu(I)Br zu und rührte die Mischung 2 h bei 140°C. Einengen der Reaktionsmischung und Chromatographie des Rückstands an Kieselgel (Cyclohexan/Ethylacetat 4/1 v/v) lieferte 0.34 Benzoxazol (4)
   ¹H-NMR (CDCl₃) ö(ppm) 7,5 (s, 1 H), 6,8 (t, 3 H), 3,9 (s, 3 H), 1;5 (s, 9 H).

### Beispiel 5: 2-Methyl-4-chlor-6-fluor-7-(4-chlor-5-difluormethoxy-1-methyl-1H-pyrazol-3-yl)benzoxazol (5)

5.1 N-Acetyl-2-brom-4-fluor-6-chlor-3-(4-chlor-5-difluormethoxy-1-methyl-1H-pyrazol-3-yl)anilin IIa.5
   In Analogie zur Herstellungsvorschrift von Beispiel 2.2 wurden 13,5 g (33 mmol) des Anilins IVa.1 aus Beispiel 2.1 in 100 ml Eisessig unter ansonsten identischen Reaktionsbedingungen umgesetzt. Durch Spaltung der primär erhaltenen Diacetylverbindung Va.5 mit 9,6 g (53 mmol) Natriummethanolat in Methanol erhielt man 7,7 g des Anilids IIa.5.
   ¹H-NMR (CDCl₃) δ(ppm) 7,3 (d, 1 H), 6,9 (br. s. 1 H), 6,7 (t, 1 H), 3,9 (s, 3 H), 2,2 (s, 3 H).
5.2 2-Methyl-4-chlor-6-fluor-7-(4-chlor-5-difluormethoxy-1-methyl-1H-pyrazol-3-yl)benzoxazol (5)
   Durch Umsetzung von 7,7 g( 17.2 mmol) Amid IIa.5 nach der in 2.3 beschriebenen Vorgehensweise erhielt man 4,28 g des Benzoxazols (5).
   ¹H-NMR (CDCl₃) δ(ppm) 7,2 (d, 1 H), 6,8 (t, 1 H), 3,9 (s, 3 H), 2,7 (s, 3 H).

### Beispiel 6: Herstellung von 2-Methoxymethyl-4-chlor-6-fluor-7-(4-chlor-5-difluormethoxy-1-methyl-1H-pyrazol-3-yl) benzoxazol (6)

6.1. N-Methoxyacetyl-2-brom-4-fluor-6-chlor-3-(4-chlor-5-difluormethoxy-1-methyl-1H-pyrazol-3-yl)-anilin IIa.69
   5,63 g (14 mmol) der Anilinverbindung IVa.1 aus Beispiel 2.1 wurden in 100 ml Tetrahydrofuran mit 3,32 g (42 mmol) Pyridin, 1,52 g Methoxyessigsäurechlorid und einer katalytischen Menge 4-Dimethylaminopyridin versetzt. Diese Mischung wurde 16 h unter Rühren zum Rückfluß erhitzt. Man entfernte die flüchtigen Bestandteile im Vakuum, nahm den Rückstand in 100 ml Ethylacetat auf und wusch die organische Phase nacheinander drei mal mit je 100 ml 2 N HCl, ein mal mit gesättigter, wässriger NaHCO₃-Lösung und trocknete über MgSO₄. Die dabei erhaltene Diacetylverbindung Va.69 (5,87 g) wurde direkt weiter umgesetzt: Man löste das Reaktionsprodukt in 50 ml Methanol, gab 3,85 g (21,4 mmol) 30 gew.-%igen Lösung von Natriummethanolat in Methanol zu und rührte 2 h bei Raumtemperatur. Anschliessend säuerte man mit 1 N HCl an, extrahierte die Mischung drei mal mit je 100 ml Methylenchlorid, wusch die vereinigten organischen Phasen einmal mit 100 ml Wasser, trocknete über MgSO₄ und chromatographiert den nach Einengen erhaltenen Rückstand an Kieselgel mit Cyclohexan/Ethylacetat (2/1 v/v). Man erhielt so 3,1 g des Anilids IIa.69.
   ¹H-NMR (CDCl₃) δ(ppm) = 8,0 (br. s 1 H), 7,3 (d, 1 H), 6,8 (t, 1 H), 4,1 (s, 2 H), 3,8 (s, 3 H), 3,6 (s, 3 H).
6.2 2-Methoxymethyl-4-chlor-6-fluor-7-(4-chlor-5-difluormethoxy-1-methyl-1H-pyrazol-3-yl)benzoxazol (6)
   Durch Umsetzung von 3,0 g (6,3 mmol) Anilid IIa.69 nach der in 2.3 beschriebenen Vorgehensweise erhielt man 0,98 g des Benzoxazols (6).
   ¹H-NMR (CDCl₃) δ(ppm) 7,3 (d, 1 H), 6,8 (t, 1 H), 4,8 (s, 2 B), 3,9 (s, 3 H), 3,5 (s, 3 H).

### Beispiel 7: Herstellung von 2-cyclopropyl-4-chlor-6-fluor-7-(4-chlor-5-trifluormethyl-1-methyl-1H-pyrazol-3-yl)benzoxazol (7)

7.1 2-Brom-4-fluor-6-chlor-3-(4-chlor-5-trifluormethyl-1-methyl-1H-pyrazol-3-yl)anilin IVc.1
   28 g (0,086 mol) 4-Fluor-6-chlor-3-(4-chlor-5-trifluormethyl-1-methyl-1H-pyrazol-3-yl)anilin (beschrieben in der EP-A 0791571) wurden in 200 ml Essigsäure gelöst, mit 35 g (0,42 mol) Natriumacetat versetzt. Hierzu tropfte man 13,7 g (0,086 mol Brom. Man rührte über Nacht, entfernte die flüchtigen Bestandteile im Vakuum und nahm den Rückstand in 200 ml Dichlormethan auf. Man wusch diese Lösung mit 2 N wässriger NaOH, trocknet über MgSO₄ und chromatographiert den nach Einengen der Lösung erhaltenen Rückstand an Kieselgel mit Cyclohexan/Ethylacetat (4/1 v/v). Man isolierte so 20 g der Titelverbindung IVc.1.
   ¹H-NMR (CDCl₃) δ(ppm) 7,2 (d, 1 H), 4,5 (br. s. 2 H), 4,0 (s, 3 H).
7.2 N-Cyclopropylcarbonyl-2-brom-4-fluor-6-chlor-3-(4-chlor-5-trifluormethyl-1-methyl-1H-pyrazol-3-yl)-anilid IIc.85
   2 g (4.9 mmol) der Anilinverbindung IVc.1 aus 7.1 wurden in 10 ml Dichlormethan und 1 ml Pyridin gelöst. Hierzu gab man eine Spatelspitze 4-Dimethylaminopyridin und 0,5 g (4,9 mmol) Cyclopropancarbonsäurechlorid. Man rührte 16 h, verdünnte mit 100 ml Wasser und extrahierte drei mal mit je 50 ml Dichlormethan. Die vereinigten organischen Phasen wurden über MgSO₄ getrocknet, und mit Cyclohexan/Ethylacetat (4/1 v/v) an Kieselgel chromatographiert.
   1. Fraktion: 1,2 g Diacylverbindung Vc.85: ¹H-NMR (CDCl₃) δ(ppm) 7,4 (d, 1 H), 4,1 (s, 3 H), 2,1 (m, 2 H), 1, 2 (m, 4 H), 0,9 (m, 4 H).
   2. Fraktion: 0.8 g Monoacylanilid IIc.85: ¹H-NMR (CDCl₃) δ(ppm) 7,3 (d, 1 H), 7,1 (br. s. 1 H), 4,1 (s 3 H), 1,6 (m 1 H), 1,2-0,8 (m, 4 H).
   Die Diacylverbindung Vc.85 löste man in 20 ml Methanol, versetzte die Lösung mit 5 ml 30 gew.%-iger Natriummethanolat-Lösung (in Methanol) und rührte 2 h. Dann versetzte man die Lösung mit 100 ml Methylenchlorid, stellte mit 10 %-iger Salzsäure auf pH 1 und trennte die organische Phase ab. Der nach Trocknen und Entfernen des Lösungsmittels erhaltene Rückstand wurde mit Fraktion 1 vereinigt. Man erhielt so 2 g der Titelverbindung IIc.85.
7.3 2-Cyclopropyl-4-chlor-6-fluor-7-(4-chlor-5-trifluormethyl-1-methyl-1H-pyrazol-3-yl)benzoxazol (7)
   2.0 g (4,2 mmol) des Anilids IIc.85 aus Stufe 7.2 wurden in 20 ml Dimethylformamid und 2 ml Pyridin mit 0,5 g (5 mmol) KHCO₃ versetzt. Man rührte 2 h bei 90 °C, gab dann 0,14 g (0,9 mmol) Cu(I)Br zu und erhitzte unter Rühren 4 h auf 140 °C. Die Reaktionsmischung wurde eingeengt und an Kieselgel mit Cyclohexan/Ethylacetat chromatographiert. Man erhielt so 1,1 g des Benzoxazols (7).
   ¹H-NMR (CDCl₃) δ(ppm) 7,2 (d, 1 H), 4,1 (s 3 H), 2,2 (m 1 H), 1,4-1,2 (m, 4 H).

### Beispiel 8: Herstellung von 2-Isopropyl-4-chlor-6-fluor-7-(4-chlor-5-trifluormethyl-1-methyl-1H-pyrazol-3-yl)benzoxazol (8)

8.1 N-Isopropylcarbonyl-2-brom-4-fluor-6-chlor-3-(4-chlor- 5-trifluormethyl-1-methyl-1H-pyrazol-3-yl)-anilid IIc.17
   Die Herstellung der Titelverbindung IIc.17 erfolgte ausgehend von Verbindung IVc.1 nach der in Beispiel 7.2 angegebenen Herstellungsvorschrift.
   ¹H-NMR (CDCl₃) δ(ppm) 7,3 (d, 1 H), 7,1 (br. s. 1 B), 4,1 (s 3 H), 2,7 (Septett 1 H), 1,3 (d, 6 H).
8.2 2-Isopropyl-4-chlor-6-fluor-7-(4-chlor-5-trifluormethyl-1-methyl-1H-pyrazol-3-yl)benzoxazol (8)
   Die Herstellung des Benzoxazols 8 erfolgte ausgehend von Verbindung IIc.17 nach der in Beispiel 7.3 angegebenen Herstellungsvorschrift.
   ¹H-NMR (CDCl₃) δ(ppm) 7,2 (d, 1 H), 4,1 (s 3 H), 3,3 (Septett 1 H), 1,3 (d, 6 H).

### Beispiel 9: Herstellung von 2-Methyl-4-chlor-6-fluor-7-(4-chlor-5-trifluormethyl-1-methyl-1H-pyrazol-3-yl)benzoxazol (9)

9.1 N-Acetyl-2-brom-4-fluor-6-chlor-3-(4-chlor- 5-trifluormethyl-1-methyl-1H-pyrazol-3-yl)-anilid IIc.5
   Die Herstellung der Titelverbindung IIc.5 erfolgte ausgehend von Verbindung IVc.1 nach der in Beispiel 7.2 angegebenen Herstellungsvorschrift.
   ¹H-NMR (CDCl₃) δ(ppm) 7,3 (d, 1 H), 7,1 (br. s. 1 H), 4,1 (s 3 H), 2,2 (s. 3 B).
9.2 2-Methyl-4-chlor-6-fluor-7-(4-chlor-5-trifluormethyl-1-methyl-1H-pyrazol-3-yl)benzoxazol (9)
   Die Herstellung des Benzoxazols 9 erfolgte ausgehend von Verbindung IIc.5 nach der in Beispiel 7.3 angegebenen Herstellungsvorschrift.
   ¹H-NMR (CDCl₃) δ(ppm) 7,2 (d, 1 H), 4,1 (s 3 H), 2,6 (s. 3 H). Herstellungsbeispiel für 2-Brom-4,6-dichlor-3-(4-brom-5-difluormethoxy-1-methyl-1H-pyrazal-3-yl)anilin
   Das in Beispiel 1.2 eingesetzte 2-Brom-4,6-dichlor-3-(4-brom-5-difluormethoxy-1-methyl-1H-pyrazol-3-yl)anilin IVa.4 wurde alternativ zur Vorschrift 1.1 ausgehend von 1,3-Dichlor-4-(5-difluormethoxy-1-methyl-1H-pyrazol-3-yl)benzol nach der im folgenden beschriebenen Synthesesequenz hergestellt:
10.1 2,4-Dichior-5-(5-difluormethoxy-1-methyl-1H-pyrazol-3-yl)nitrobenzol
   3.0 g (10,2 mmol) 1,3-Dichlor-4-(5-difluormethoxy-1-methyl-1H-pyrazol-3-yl)benzol wurden in 10 ml konzentrierter Schwefelsäure gelöst. Hierzu tropfte man bei 0°C 0,7 g (11,2 mmol) 100-%ige Salpetersäure und rührte dann 1 h bei 0°C bis 10°C. Dann goss man die Reaktionsmischung auf Eis, saugte den Niederschlag ab, wusch mit 100 ml Wasser nach und trocknete. Man erhielt so 3,24 g des Nitrobenzols als gelben Feststoff mit einem Schmelzpunkt von 134 bis 137°C.
   ¹H-NMR (CDCl₃) δ(ppm) 8,4 (s. 1H), 7,6 (s. 1H), 6,6 (t. 1H), 6,4 (s. 1H), 3,8 (s. 3H).
10.2 2,4-Diehlor-5-(5-difluormethoxy-1-methyl-1H-pyrazol-3-yl)anilin
   1,5 g (4,2 mmol) 2,4-Dichlor-5-(5-difluormethoxy-1-methyl-1H-pyrazol-3-yl)nitrobenzol wurden in einer Mischung aus 50 ml Tetrahydrofuran und 50 ml Methanol gelöst und mit 4 g Raney-Nickel versetzt. Die Mischung wurde bei einem Wasserstoffüberdruck von 0,3 bar 4 h bei Raumtemperatur gerührt und anschliessend über Kieselgur filtriert. Das Filtrat wurde mit Magnesiumsulfat getrocknet und anschliessend zur Trockne eingeengt. Man erhielt so 1,3 g des Anilins.
   ¹H-NMR (CDCl₃) δ(ppm) 7,4-7,1 (m. 2H), 6,6 (t. 1H), 6,4 (s. 1H), 4,1 (br. 2H), 3,8 (s. 3H).
10.3 2-Brom-4,6-dichlor-3-(5-difluormethoxy-1-methyl-1H-pyrazol-3-yl)anilin IVa.4
   1 g (2,9 mmol) 2,4-Dichlor-5-(5-difluormethoxy-1-methyl-1H-pyrazol-3-yl)anilin und 2,4 g (29 mmol) Natriumacetat legte man in 100 ml Eisessig vor und tropfte hierzu bei Raumtemperatur 0,93 g (5,6 mmol) Brom. Man rührte über Nacht, engte die Reaktionsmischung ein, nahm den Rückstand in Toluol auf und wusch mit 50 ml 5 gew.-%iger wässriger Natronlauge neutral. Die organische Phase wurde über Magnesiumsulfat getrocknet und eingeengt. Man erhielt so 1,3 g des Anilins IVa.4. Das erhaltene Produkt war mit der nach Beispiels 1.1 erhaltenen Verbindung identisch.

## Patentansprüche

1. verfahren zur Herstellung von 7-(Pyrazol-3-yl)-benzoxazolen der allgemeinen Formel I 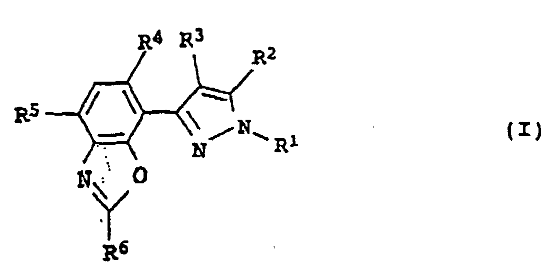 in der die Variablen R¹ - R⁶ folgende Bedeutungen haben:
R¹ Wasserstoff, C₁-C₄-Alkyl oder C₁-C₄-Halogenalkyl;
R² cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
R³ Wasserstoff, Halogen, Cyano, C₁-C₄-Alkyl oder C₁-C₄-Halogenalkyl;
R⁴ Halogen;
R⁵ Fluor, Chlor oder Cyano;
R⁶ Wasserstoff, C₁-C₆-Alkyl, C₁-C₆-Halogenalkyl, C₂-C₆-Alkenyl, C₂-C₆-Halogenalkenyl, C₂-C₆-Alkinyl, C₁-C₄-Alkoxy-C₁-C₄-alkyl, C₁-C₄-Cyanoalkyl, C₁-C₄-Alkylthio-C₁-C₄-alkyl, (C₁-C₄-Alkoxy)carbonyl-C₁-C₄-alkyl, C₃-C₈-Cycloalkyl, C₃-C₈-Cycloalkyl-C₁-C₄-alkyl, C₃-C₈-Cycloalkyloxy-C₁-C₄-alkyl, Phenyl, Phenyl-C₁-C₄-alkyl, 4- bis 7-gliedriges Heterocyclyl oder Heterocyclyl-C₁-C₄-alkyl, wobei jeder cycloalkyl-, Phenyl- und jeder Heterocyclyl-Ring unsubstituiert sein oder ein, zwei oder drei Substituenten tragen kann, die unabhängig voneinander ausgewählt sind unter Cyano, Nitro, Amino, Hydroxy, Carboxy, Halogen, C₁-C₄-Alkyl, C₁-C₄-Halogenalkyl, C₁-C₄-Alkoxy, C₁-C₄-Halogenalkoxy, C₁-C₄-Alkylthio;
**dadurch gekennzeichnet, daß** man ein 2-Halogen-3-(pyrazol-3-yl)anilid der allgemeinen Formel II. 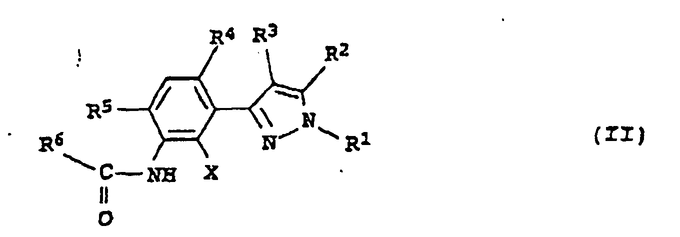 in der die Variablen R¹ - R⁶ die zuvor angegebene Bedeutung haben und X für Brom oder Iod steht, in Gegenwart einer Kupfer (I) - Verbindung und einer Base zu einer Verbindung der allgemeinen Formel I umsetzt, wobei das Molverhältnis von Kupfer (I)-Verbindung zu eingesetzter Verbindung II < 1:1 ist.

2. Verfahren nach Anspruch 1 **dadurch gekennzeichnet, dass** das Molverhältnis von Kupfer (I) - Verbindung zu eingesetzter Verbindung II im Bereich von 0,05:1 bis 0,8:1 liegt.

3. Verfahren nach einem der vorhergehenden Ansprüche, **dadurch gekennzeichnet, dass** die Base ausgewählt ist unter den Alko holaten, Amiden, Hydriden, Hydroxiden, Hydrogencarbonaten und Carbonaten von Alkalimetallen und von Erdalkalimetallen.

4. Verfahren nach einem der vorhergehenden Ansprüche, **dadurch gekennzeichnet, dass** man eine wenigstens equimolare Menge Base, bezogen auf die Verbindung II einsetzt.

5. Verfahren nach einem der vorhergehenden Ansprüche, **dadurch gekennzeichnet, dass** man die Umsetzung von Verbindung II zur Verbindung I in einem polar aprotischen Lösungsmittel oder Lösungsmittelgemisch durchführt, wobei das Lösungsmittel ausgewählt ist unter Dimethylformamid (DMF), Dimethylsulfoxid (DMSO), N-Methylpyrrolidon (NMP), N,N-Dimethylacetamid (DMA), Acetonitril, Propionitril, Pyridin und den Dimethyldi- oder - triethylenglykolen und deren Gemischen.

6. Verfahren nach Anspruch 1, dadurch gekennzeichnt, dass man eine Verbinduhg II umsetzt, worin die Variablen R¹ - R⁶ die folgenden Bedeutungen aufweisen:
R¹ Wasserstoff, Methyl oder Ethyl;
R² Cyano, Difluormethoxy, Trifluormethyl oder Methylsulfonyl;
R³ Halogen;
R⁴ Halogen;
R⁵ Fluor, Chlor oder Cyano;
R⁶ Wasserstoff, C₁-C₄-Alkyl, C₁-C₄-Halogenalkyl, C₂-C₄-Alkenyl, C₂-C₄-Halogenalkenyl, C₂-C₄-Alkinyl, C₁-C₄-Alkoxy-C₁-C₄-alkyl, C₁-C₄-Alkoxycarbonyl-C₁-C₄-alkyl, C₃-C₈-Cycloalkyl, C₃-C₈-Cycloalkyl-C₁-C₄-alkyl, Phenyl, Phenyl-C₁-C₄-alkyl, 4- bis 7-gliedriges Heterocyclyl oder Heterocyclyl-C₁-C₄-alkyl, wobei der Phenylring, der Cycloalkylring und der Heterocyclylring unsubstituiert sein können oder einen oder zwei Substituenten, ausgewählt unter Cyano, Halogen, C₁-C₄-Alkyl, C₁-C₄-Halogenalkyl und C₁-C₄-Alkoxy tragen können.

7. Verfahren nach Anspruch 1, **dadurch gekennzeichnet, dass** es zusätzlich die folgenden Verfahrenschritte zur Herstellung der Verbindung II umfasst:
i. Halogenierung eines 3-(Pyrazol-3-yl)anilins der Formel III 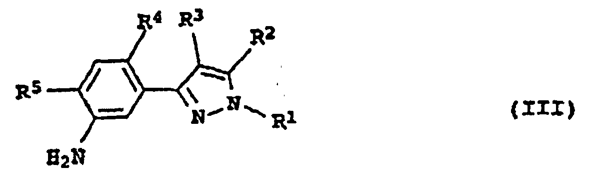 **zu einem 2-Halogen-3-(pyrazol-3-yl)anilin der Formel IV,** 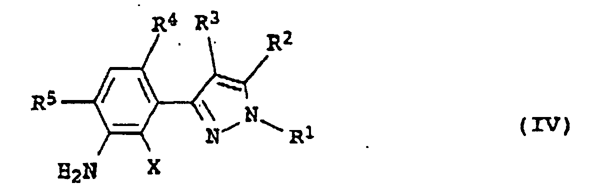
ii. Umsetzung des 3-Halogen-2-(pyrazol-3-yl)anilins IV mit einem Acylierungsmittel der Formel R⁶-C(O)-Y, worin Y für eine Abgangsgruppe steht, zu einem Anilid der Formel II und/oder einer Diacylverbindung der Formel V 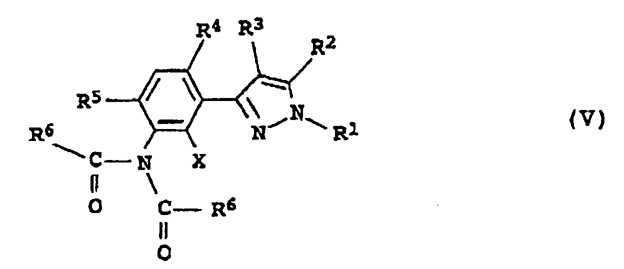
iii. gegebenenfalls partielle solvolyse der Verbindung V zu dem Anilid der Formel II,
wobei in den Verbindungen der Formeln III, IV und V die Variablen R¹ - R⁶ und X die zuvor genannten Bedeutungen aufweisen.

8. Verfahren nach Anspruch 7, **dadurch gekennzeichnet, dass** man zur Herstellung von Verbindungen II-A, worin R³ und X für Brom stehen, in Schritt i ein 3-(Pyrazol-3-yl)anilin der Formel III-A, 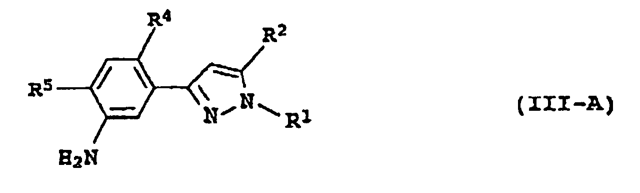 zu einem 2-Brom-3-(4-brompyrazol-3-yl)anilin der Formel IV-A, 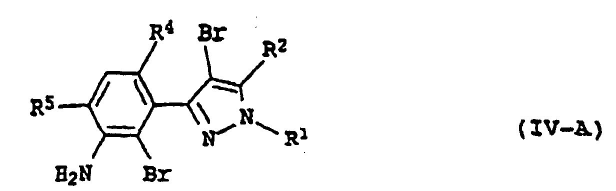 bromiert, Wobei in den Verbindungen der Formeln III-A und IV-A die Variablen R¹, R², R⁴, R⁵ die zuvor genannten Bedeutungen aufweisen.

9. Verfahren nach Anspruch 8, **dadurch gekennzeichnet, dass** es zusätzlich die folgenden Verfahrenschritte zur Herstellung von Verbindungen III-A umfasst:
a. Nitrierung eines 3-(Pyrazol-3-yl)benzols der Formal VI-A, 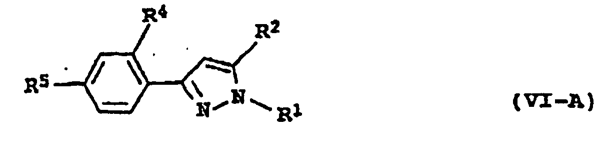 worin die Variablen R¹, R², R⁴ und R⁵ die zuvor genannten Bedeutungen aufweisen, zu einem ein 3-(Pyrazol-3-yl)-1-nitrobenzol VII-A und 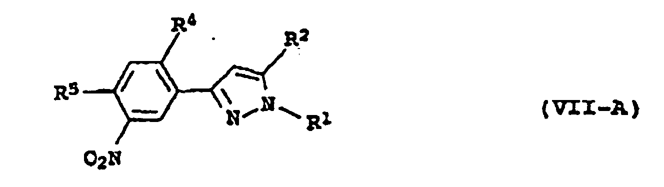
b. Umsetzung von VII-A mit einem Reduktionsmittel zu einem ein 3-(Pyrazol-3-yl)anilin der Formel III-A gemäss Anspruch 8.

10. Verbindungen der allgemeinen Formeln II, IV und V wie in Anspruch 7 definiert und deren landwirtschaftlich verträgliche Salze.

11. Verbindungen nach Anspruch 10, worin die Variablen R¹ - R⁶ die folgenden Bedeutungen aufweisen:
R¹ Wasserstoff, Methyl oder Ethyl;
R² Cyano, Difluormethoxy, Trifluormethyl oder Methylsulfonyl;
R³ Halogen;
R⁴ Halogen;
R⁵ Fluor, Chlor oder Cyano;
R⁶ Wasserstoff, C₁-C₄-Alkyl, C₁-C₄-Halogenalkyl, C₂-C₄-Alkenyl, C₂-C₄-Halogenalkenyl, C₂-C₄-Alkinyl, C₁-C₄-Alkoxy-C₁-C₄-alkyl, C₁-C₄-Alkoxycarbonyl-C₁-C₄-alkyl, C₃-C₈-Cycloalkyl, C₃-C₈-Cycloalkyl-C₁-C₄-alkyl, Phenyl, Phenpl-C₁-C₄-alkyl, 4- bis 7-gliedriges Heterocyclyl oder Heterocyclyl-C₁-C₄-alkyl, wobei der Phenylring, der Cycloalkylring und der Heterocyclylring unsubstituiert sein können oder einen oder zwei Substituenten, ausgewählt unter Cyano, Halogen, C₁-C₄-Alkyl, C₁-C₄-Halogenalkyl und C₁-C₄-Alkoxy tragen können.

12. Verbindungen nach Anspruch 10 oder 11, worin die Variable X für Brom steht.

13. Verbindungen nach Anspruch 10, 11 oder 12, worin die Variable R² für Difluormethoxy steht.

## Claims

1. A process for preparing 7-(pyrazol-3-yl)benzoxazoles of the formula I 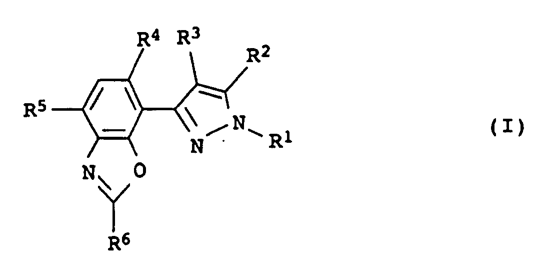 in which the variables R¹ - R⁶ are as defined below:
R¹ is hydrogen, C₁-C₄-alkyl or C₁-C₄-haloalkyl;
R² is cyano, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylsulf inyl, C₁-C₄-haloalkylsulfinyl, C₁-C₄-alkylsulfonyl or C₁-C₄-haloalkylsulfonyl;
R³ is hydrogen, halogen, cyano, C₁-C₄-alkyl or C₁-C₄-haloalkyl;
R⁴ is halogen;
R⁵ is fluorine, chlorine or cyano;
R⁶ is hydrogen, C₁-C₆-alkyl, C₁-C₆-haloalkyl, C₂-C₆-alkenyl, C₂-C₆-haloalkenyl, C₂-C₆-alkynyl, C₁-C₄-alkoxy-C₁-C₄-alkyl, C₁-C₄-cyanoalkyl, C₁-C₄-alkylthio-C₁-C₄-alkyl, (C₁-C₄-alkoxy)carbonyl-C₁-C₄-alkyl, C₃-C₈-cycloalkyl, C₃-C₈-cycloalkyl-C₁-C₄-alkyl, C₃-C₈-cycloalkyloxy-C₁-C₄-alkyl, phenyl, phenyl-C₁-C₄-alkyl, 4- to 7-membered heterocyclyl or heterocyclyl-C₁-C₄-alkyl, where each cycloalkyl, phenyl and heterocyclyl ring can be unsubstituted or may carry one, two or three substituents selected independently of one another from the group consisting of cyano, nitro, amino, hydroxyl, carboxyl, halogen, C₁-C₄-alkyl, C₁-C₄-haloalkyl, C₁-C₄-alkoxy, C₁-C₄-haloalkoxy, C₁-C₄-alkylthio;
which comprises reacting a 2-halo-3-(pyrazol-3-yl)anilide of the formula II 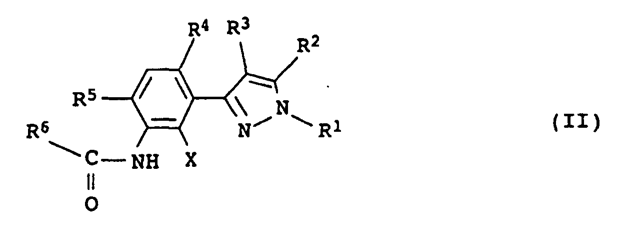 in which the variables R¹ - R⁶ are as defined above and X is bromine or iodine in the presence of a copper(I) compound and a base to give a compound of the formula I, wherein the molar ratio of copper(I) compound to the compound II used is < 1:1.

2. A process as claimed in claim 1, wherein the molar ratio of copper(I) compound to the compound II used is in the range from 0.05:1 to 0.8:1.

3. A process as claimed in any of the preceding claims, wherein the base is selected from the alkoxides, amides, hydrides, hydroxides, bicarbonates and carbonates of alkali metals and of alkaline earth metals.

4. A process as claimed in any of the preceding claims, wherein an at least equimolar amount of base, based on the compound II, is employed.

5. A process as claimed in any of the preceding claims, wherein the conversion of compound II into compound I is carried out in a polar aprotic solvent or solvent mixture, where the solvent is selected from the group consisting of dimethylformamide (DMF), dimethyl sulfoxide (DMSO), N-methylpyrrolidone (NMP), N,N-dimethylacetamide (DMA), acetonitrile, propionitrile, pyridine and dimethyldi- or - triethylene glycols and mixtures thereof.

6. A process as claimed in claim 1, wherein a compound II is reacted in which the variables R¹ - R⁶ are as defined below:
R¹ is hydrogen, methyl or ethyl;
R² is cyano, difluoromethoxy, trifluoromethyl or methylsulfonyl;
R³ is halogen;
R⁴ is halogen;
R⁵ is fluorine, chlorine or cyano;
R⁶ is hydrogen, C₁-C₄-alkyl, C₁-C₄-haloalkyl, C₂-C₄-alkenyl, C₂-C₄-haloalkenyl, C₂-C₄-alkynyl, C₁-C₄-alkoxy-C₁-C₄-alkyl, C₁-C₄-alkoxycarbonyl-C₁-C₄-alkyl, C₃-C₈-cycloalkyl, C₃-C₈-cycloalkyl-C₁-C₄-alkyl, phenyl, phenyl-C₁-C₄-alkyl, 4- to 7-membered heterocyclyl or heterocyclyl-C₁-C₄-alkyl, where the phenyl ring, the cycloalkyl ring and the heterocyclyl ring can be unsubstituted or may carry one or two substituents, selected from the group consisting of cyano, halogen, C₁-C₄-alkyl, C₁-C₄-haloalkyl and C₁-C₄-alkoxy.

7. A process as claimed in claim 1, which additionally comprises the following process steps for preparing the compound II:
i. halogenation of a 3-(pyrazol-3-yl)aniline of the formula III 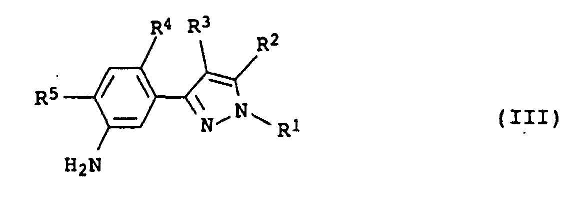 to give a 2-halo-3-(pyrazol-3-yl)aniline of the formula IV, 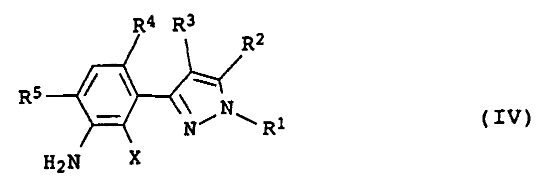
ii. reaction of the 3-halo-2-(pyrazol-3-yl)aniline IV with an acylating agent of the formula R⁶-C(O)-Y, in which Y is a leaving group, to give an anilide of the formula II and/or a diacyl compound of the formula V 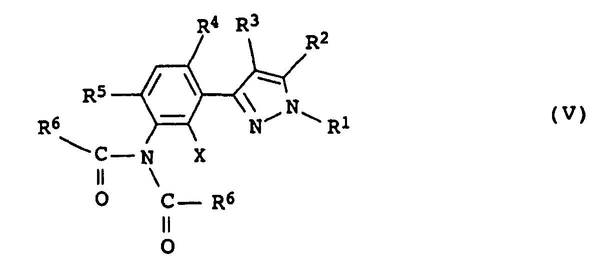
iii. if appropriate, partial solvolysis of the compound V to give the anilide of the formula II,
where the variables R¹ - R⁶ and X in the compounds of the formulae III, IV and V are as defined above.

8. A process as claimed in claim 7, wherein, for preparing compounds II-A in which R³ and X are bromine, in step i a 3-(pyrazol-3-yl)aniline of the formula III-A, 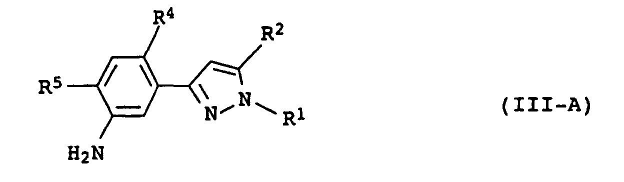 is brominated to give a 2-bromo-3-(4-bromopyrazol-3-yl)-aniline of the formula IV-A, 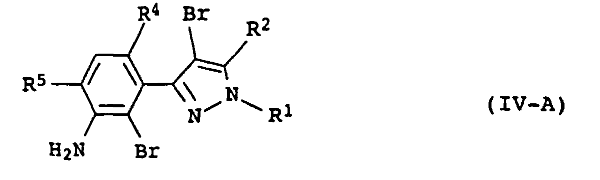 where the variables R¹, R², R⁴ and R⁵ in the compounds of the formulae III-A and IV-A are as defined above.

9. A process as claimed in claim 8, which additionally comprises the following process steps for preparing compounds III-A:
a. nitration of a 3-(pyrazol-3-yl)benzene of the formula VI-A, 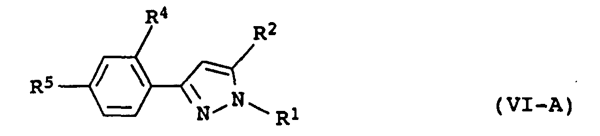 in which the variables R¹, R², R⁴ and R⁵ are as defined above to give a 3-(pyrazol-3-yl)-1-nitrobenzene VII-A and 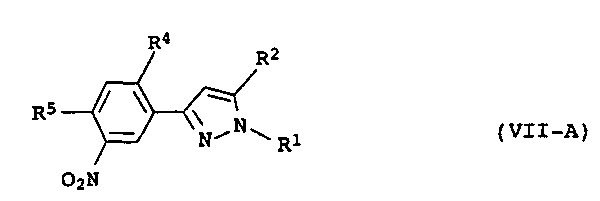
b. reaction of VII-A with a reducing agent to give a 3-(pyrazol-3-yl)aniline of the formula III-A as claimed in claim 8.

10. A compound of the formula II, IV or V as defined in claim 7 and its agriculturally acceptable salts.

11. A compound as claimed in claim 10 in which the variables R¹-R⁶ are as defined below:
R¹ is hydrogen, methyl or ethyl;
R² is cyano, difluoromethoxy, trifluoromethyl or methylsulfonyl;
R³ is halogen;
R⁴ is halogen;
R⁵ is fluorine, chlorine or cyano;
R⁶ is hydrogen, C₁-C₄-alkyl, C₁-C₄-haloalkyl, C₂-C₄-alkenyl, C₂-C₄-haloalkenyl, C₂-C₄-alkynyl, C₁-C₄-alkoxy-C₁-C₄-alkyl, C₁-C₄-alkoxycarbonyl-C₁-C₄-alkyl, C₃-C₈-cycloalkyl, C₃-C₈-cycloalkyl-C₁-C₄-alkyl, phenyl, phenyl-C₁-C₄-alkyl, 4- to 7-membered heterocyclyl or heterocyclyl-C₁-C₄-alkyl, where the phenyl ring, the cycloalkyl ring and the heterocyclyl ring may be ' unsubstituted or may carry one or two substituents selected from the group consisting of cyano, halogen, C₁-C₄-alkyl, C₁-C₄-haloalkyl and C₁-C₄-alkoxy.

12. A compound as claimed in claim 10 or 11 in which the variable X is bromine.

13. A compound as claimed in claim 10, 11 or 12 in which the variable R² is difluoromethoxy.

## Revendications

1. Procédé pour la préparation de 7-(pyrazol-3-yl)-benzoxazoles répondant à la formule générale I 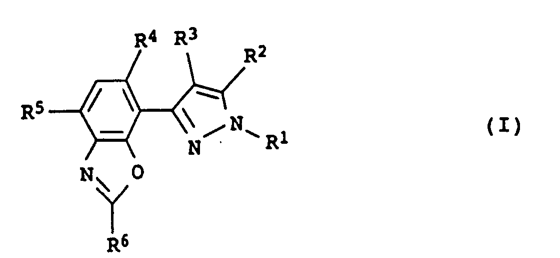 dans laquelle les variables R¹ à R⁶ ont les significations suivantes :
R' représente un atome d'hydrogène, un groupe alkyle en C₁-C₄ ou un groupe halogénalkyle en C₁-C₄ ;
R² représente un groupe cyano, un groupe alkyle en C₁-C₄, un groupe halogénalkyle en C₁-C₄, un groupe alcoxy en C₁-C₄, un groupe halogénalcoxy en C₁-C₄, un groupe alkyl(en C₁-C₄)thio, un groupe halogénalkyl(en C₁-C₄)thio, un groupe alkyl(en C₁-C₄)sulfinyle, un groupe halogénalkyl(en C₁-C₄)sulfinyle, un groupe alkyl(en C₁-C₄)sulfonyle ou un groupe halogénalkyl(en C₁-C₄)sulfonyle ;
R³ représente un atome d'hydrogène, un atome d'halogène, un groupe cyano, un groupe alkyle en C₁-C₄ ou un groupe halogénalkyle en C₁-C₄ ;
R⁴ représente un atome d'halogène;
R⁵ représente un atome de fluor, un atome de chlore où un groupe cyano ;
R⁶ représente un atome d'hydrogène, un groupe alkyle en C₁-C₆, un groupe halogénalkyle en C₁-C₆, un groupe alcényle en C₂-C₆, un groupe halogénalcényle en C₂-C₆, un groupe alcynyle en C₂-C₆, un groupe alcoxy(en C₁-C₄)alkyle en C₁-C₄, un groupe cyanoalkyle en C₁-C₄, un groupe alkyl(en C₁-C₄)thioalkyle en C₁-C₄, un groupe (alcoxy en C₁-C₄)carbonylalkyle en C₁-C₄, un groupe cycloalkyle en C₃-C₈, un groupe cycloalkyl(en C₃-C₈)alkyle en C₁-C₄, un groupe cycloalkyl(en C₃-C₈)oxyalkyle en C₁-C₄, un groupe phényle, un groupe phénylalkyle en C₁-C₄, un groupe hétérocyclyle ou un groupe hétérocyclylalkyle en C₁-C₄ de 4 à 7 membres, chaque noyau cycloalkyle, chaque noyau phényle et chaque noyau hétérocyclyle pouvant être non substitué ou pouvant porter un, deux ou trois substituants choisis, indépendamment l'un de l'autre, parmi le groupe comprenant un groupe cyano, un groupe nitro, un groupe amino, un groupe hydroxyle, un groupe carboxyle, un atome d'halogène, un groupe alkyle en C₁-C₄, un groupe halogénalkyle en C₁-C₄, un groupe alcoxy en C₁-C₄, un groupe halogénalcoxy en C₁-C₄, un groupe alkyl(en C₁-C₄)thio ;
**caractérisé en ce qu'**on fait réagir un 2-halogéno-3-(pyrazol-3-yl)-anilide répondant à la formule générale II 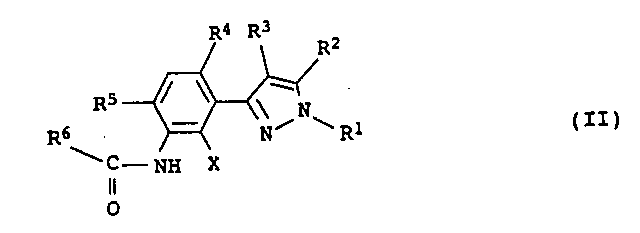 dans laquelle les variables R¹ à R⁶ ont les significations indiquées ci-dessus et X représente un atome de brome ou un atome d'iode, en présence d'un composé de cuivre(I) et d'une base pour obtenir un composé répondant à la formule générale I, le rapport molaire du composé de cuivre(I) au composé II mis en oeuvre étant inférieur à 1 : 1.

2. Procédé selon la revendication 1, **caractérisé en ce que** le rapport molaire du composé de cuivre(I) au composé II mis en oeuvre se situe dans la plage de 0,05 : 1 à 0,8 : 1.

3. Procédé selon l'une quelconque des revendications précédentes, **caractérisé en ce que** la base est choisie parmi le groupe comprenant des alcoolates, des amides, des hydrures, des hydroxydes, des hydrogénocarbonates et des carbonates de métaux alcalins et de métaux alcalino-terreux.

4. Procédé selon l'une quelconque des revendications précédentes, **caractérisé en ce qu'**on met en oeuvre une quantité au moins équimolaire de la base, rapportée au composé II.

5. Procédé selon l'une quelconque des revendications précédentes, **caractérisé en ce qu'**on effectue la mise en réaction du composé II pour obtenir le composé 1 dans un solvant aprotique polaire ou dans un mélange de solvants aprotiques polaires, le solvant étant choisi parmi le groupe comprenant le diméthylformamide (DMF), le diméthylsulfoxyde (DMSO), la N-méthylpyrrolidone (NMP), le N,N-diméthylacétamide (DMA), l'acétonitrile, le propionitrile, la pyridine, et les diméthyldiéthylèneglycols ou les diméthyltriéthylèneglycols, et leurs mélanges.

6. Procédé selon la revendication 1, **caractérisé en ce qu'**on fait réagir un composé II, les variables R¹ à R⁶ présentant les significations suivantes :
R¹ représente un atome d'hydrogène, un groupe méthyle ou un groupe éthyle ;
R² représente un groupe cyano, un groupe difluorométhoxy, un groupe trifluorométhyle ou un groupe méthylsulfonyle ;
R³ représente un atome d'halogène ;
R⁴ représente un atome d'halogène ;
R⁵ représente un atome de fluor, un atome de chlore ou un groupe cyano ;
R⁶ représente un atome d'hydrogène, un groupe alkyle en C₁-C₄, un groupe halogénalkyle en C₁-C₄, un groupe alcényle en C₂-C₄, un groupe halogénalcényle en C₂-C₄, un groupe alcynyle en C₂-C₄, un groupe alcoxy(en C₁-C₄)alkyle en C₁-C₄, un groupe alcoxy(en C₁-C₄)carbonylalkyle en C₁-C₄, un groupe cycloalkyle en C₃-C₈, un groupe cycloalkyl(en C₃-C₈)alkyle en C₁-C₄, un groupe phényle, un groupe phénylalkyle en C₁-C₄, un groupe hétérocyclyle ou un groupe hétérocyclylalkyle en C₁-C₄ de 4 à 7 membres, le noyau phényle, le noyau cycloalkyle et le noyau hétérocyclyle pouvant être non substitués ou pouvant porter un ou deux substituants choisis parmi le groupe comprenant un groupe cyano, un atome d'halogène, un groupe alkyle en C₁-C₄, un groupe halogénalkyle en C₁-C₄ et un groupe alcoxy en C₁-C₄.

7. Procédé selon la revendication 1, **caractérisé en ce qu'**il comprend en outre les étapes opératoires ci-après, pour la préparation du composé II :
i. l'halogénation d'une 3-(pyrazol-3-yl)aniline répondant à la formule III 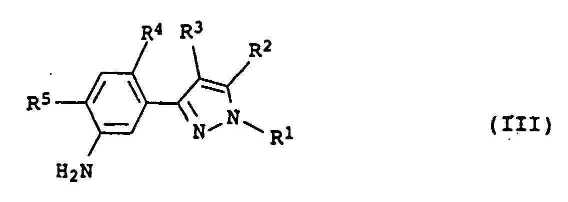 pour obtenir une 2-halogéno-3-(pyrazol-3-yl)aniline répondant à la formule IV 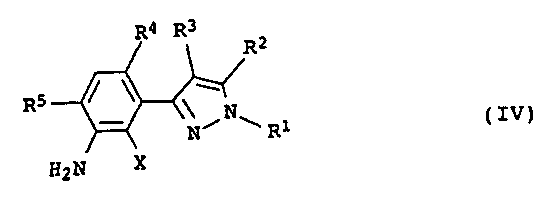
ii. la mise en réaction de la 3-halogéno-2-(pyrazol-3-yl)aniline IV avec un agent d'acylation répondant à la formule R⁶-C(O)-Y dans laquelle Y représente un groupe sortant, pour obtenir un anilide répondant à la formule II et/ou un composé diacylé répondant à la formule V 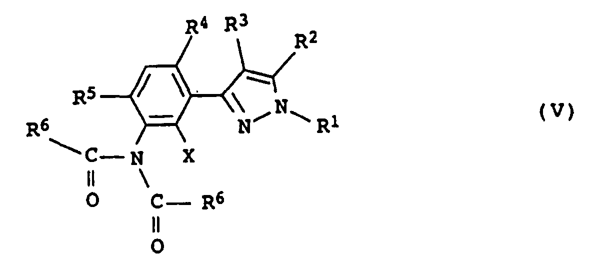
iii. le cas échéant, la solvolyse partielle du composé V pour obtenir l'anilide répondant à la formule II,
dans les composés répondant aux formules III, IV et V, les variables R¹ à R⁶ et X présentant les significations mentionnées ci-dessus.

8. Procédé selon la revendication 7, **caractérisé en ce que**, pour la préparation des composés II-A dans lesquels R³ et X représentent un atome de brome, à l'étape i, on soumet à une bromation une 3-(pyrazol-3-yl)aniline répondant à la formule III-A, 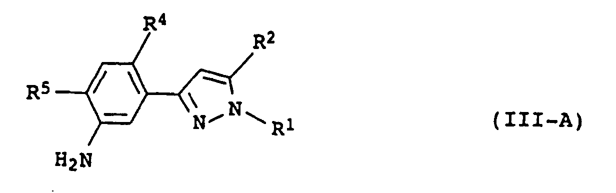 pour obtenir une 2-bromo-3-(4-bromopyrazol-3-yl)aniline répondant à la formule IV-A, 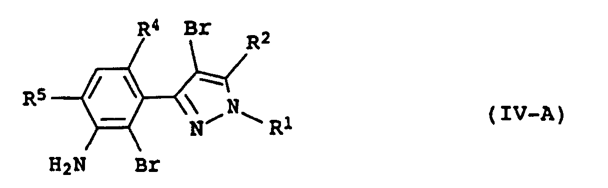 dans les composés répondant aux formules III-A et IV-A, les variables R¹, R², R⁴, R⁵ présentant les significations indiquées ci-dessus.

9. Procédé selon la revendication 8, **caractérisé en ce qu'**il comprend, en outre, les étapes opératoires ci-après, pour la préparation des composés III-A :
a) la nitration d'un 3-(pyrazol-3-yl) benzène répondant à la formule VI-A, 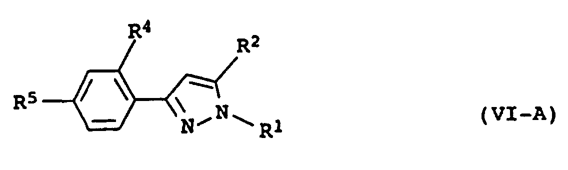 dans laquelle les variables R¹, R², R⁴ et R⁵ présentent les significations indiquées ci-dessus, pour obtenir un 3-(pyrazol-3-yl)-1-nitrobenzène VII-A, et 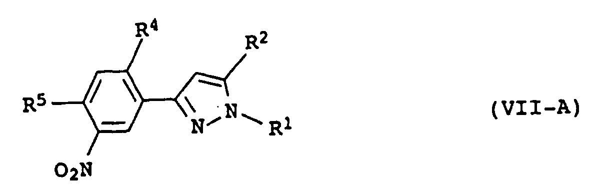
b) la mise en réaction de VII-A avec un agent de réduction pour obtenir une 3-(pyrazol-3-yl)aniline répondant à la formule III-A selon la revendication 8.

10. Composés répondant aux formules générales II, IV et V, comme défini à la revendication 7, ainsi que leurs sels acceptables du point de vue agricole.

11. Composés selon la revendication 10, dans lesquels les variables R¹ à R⁶ présentent les significations suivantes :
R¹ représente un atome d'hydrogène, un groupe méthyle ou un groupe éthyle ;
R² représente un groupe cyano, un groupe difluorométhoxy, un groupe trifluorométhyle ou un groupe méthylsulfonyle ;
R³ représente un atome d'halogène ;
R⁴ représente un atome d'halogène;
R⁵ représente un atome de fluor, un atome de chlore ou un groupe cyano ;
R⁶ représente un atome d'hydrogène, un groupe alkyle en C₁-C₄, un groupe halogénalkyle en C₁-C₄, un groupe alcényle en C₂-C₄, un groupe halogénalcényle en C₂-C₄, un groupe alcynyle en C₂-C₄, un groupe alcoxy(en C₁-C₄)alkyle en C₁-C₄, un groupe alcoxy(en C₁-C₄)carbonylalkyle en C₁-C₄, un groupe cycloalkyle en C₃-C₈, un groupe cycloalkyl(en C₃-C₈)alkyle en C₁-C₄, un groupe phényle, un groupe phénylalkyle en C₁-C₄, un groupe hétérocyclyle ou un groupe hétérocyclylalkyle en C₁-C₄ de 4 à 7 membres, le noyau phényle, le noyau cycloalkyle et le noyau hétérocyclyle pouvant être non substitués ou pouvant porter un ou deux substituants choisis parmi le groupe comprenant un groupe cyano, un atome d'halogène, un groupe alkyle en C₁-C₄, un groupe halogénalkyle en C₁-C₄ et un groupe alcoxy en C₁-C₄.

12. Composés selon la revendication 10 ou 11, dans lesquels la variable X représente un atome de brome.

13. Composés selon la revendication 10, 11 ou 12, dans lesquels la variable R² représente un groupe difluorométhoxy.
